(19) Europäisches Patentamt
European Patent Office
Office européen des brevets

(11) EP 0 563 128 B1

(12) EUROPÄISCHE PATENTSCHRIFT

(45) Veröffentlichungstag und Bekanntmachung des Hinweises auf die Patenterteilung: **24.03.1999 Patentblatt 1999/12**

(21) Anmeldenummer: **92901178.1**

(22) Anmeldetag: **20.12.1991**

(51) Int. Cl.$^6$: **A61K 31/445**, A61K 31/495, A61K 31/54, A61K 31/535, A61K 31/475, A61K 31/47

(86) Internationale Anmeldenummer: **PCT/EP91/02470**

(87) Internationale Veröffentlichungsnummer: **WO 92/11010 (09.07.1992 Gazette 1992/17)**

# (54) NEUE 3,4-DIHYDROISOCHINOLINDERIVATE UND NEUE PHARMAZEUTISCHE VERWENDUNG CARBOCYCLISCH UND HETEROCYCLISCH ANELLIERTER DIHYDROPYRIDINE

NEW 3,4-DIHYDROISOQUINOLINE DERIVATES AND NEW PHARMACEUTICAL USE OF CARBOCYCLICALLY AND HETEROCYCLICALLY ANNULATED DIHYDROPYRIDINES

NOUVEAUX DERIVES DE 3,4-DIHYDROISOQUINOLEINE ET UTILISATION PHARMACEUTIQUE NOUVELLE DE DIHYDROPYRIDINES CARBOCYCLIQUEMENT ET HETEROCYCLIQUEMENT CYCLISEES

(84) Benannte Vertragsstaaten:
**AT BE CH DE DK ES FR GB GR IT LI LU NL SE**

(30) Priorität: **22.12.1990 DE 4041482**

(43) Veröffentlichungstag der Anmeldung: **06.10.1993 Patentblatt 1993/40**

(60) Teilanmeldung: **96119881.9 / 0 781 556**

(73) Patentinhaber:
- **Boehringer Ingelheim Pharma KG**
  **55216 Ingelheim am Rhein (DE)**
  Benannte Vertragsstaaten:
  **BE CH DE DK ES FR GR IT LI LU NL SE AT**
- **BOEHRINGER INGELHEIM INTERNATIONAL GmbH**
  **55218 Ingelheim am Rhein (DE)**
  Benannte Vertragsstaaten:
  **GB**

(72) Erfinder:
- **LÖSEL, Walter**
  **D-6535 Gau-Algesheim (DE)**
- **ROOS, Otto**
  **D-6501 Schabenheim (DE)**
- **ARNDTS, Dietrich**
  **D-6531 Appenheim (DE)**
- **KUHN, Franz, Josef**
  **D-6535 Gau-Algesheim (DE)**
- **STRELLER, Ilse**
  **D-6534 Stromberg (DE)**

(74) Vertreter: **Laudien, Dieter, Dr.**
**Boehringer Ingelheim GmbH,**
**Binger Strasse 173**
**55216 Ingelheim (DE)**

(56) Entgegenhaltungen:
**EP-A- 0 251 194** **DE-A- 3 621 413**
**DE-A- 3 827 727**

- **Stroke, Band 17, Nr. 4, 1986, A. FUJISAWA et al.: "The effect of the calcium antagonist nimodipine on the gerbil model of experimental cerebral ischemia", S. 748-752, siehe Absätze "Introduction" und "Discussion"**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 563 128 B1

Printed by Xerox (UK) Business Services
2.16.7/3.6

## Beschreibung

Aus der EP-A 37 934 sind Dihydroisochinoline der allgemeinen Formel Ia bekannt geworden. Die dort genannten Verbindungen sind cardioton wirksam und weisen eine kontraktilitätssteigernde und blutdruckbeeinflussende Wirkkomponente auf. Sie sind zur Verbesserung der Gewebsdurchblutung und der Gewebssauerstoffversorgung vorgeschlagen worden. Diese Verwendungsmöglichkeiten beruhen auf der vaskulären Wirksamkeit der Verbindungen. In der EP-A 251 194 ist beschrieben worden, daß carbocyclisch und heterocyclisch annelierte Dihydropyridine cardioprotektive Wirkung besitzen und einen völlig neuen Typ Ca-antagonistischer Verbindungen verkörpern.

Gegenstand der vorliegenden Erfindung ist die Verwendung der aus der genannten EP-A 251 194 bekannten Verbindungen als hirnprotektive Mittel, insbesondere bei der Behandlung von Patienten, die einen Schlaganfall erlitten haben oder gefährdet sind, einen Schlaganfall zu erleiden. Ein weiterer Gegenstand der Erfindung ist eine Gruppe von neuen annelierten Dihydropyridinen, nämlich vom neuen 3,4-Dihydroisochinolinderivaten der weiter unten definierten allgemeinen Formel Ie. Diese neuen Verbindungen sind für die Verwendung als hirnprotektive Mittel geeignet.

Die Erfindung betrifft somit die Verwendung von carbocyclisch und heterocyclisch anellierten Dihydropyridinen der Formel

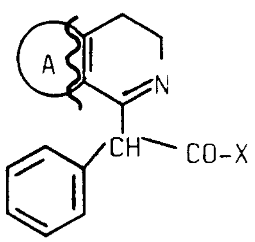

I

worunter auch deren tautomere Formen der Formel II erfaßt werden, wobei diese Bezeichnung zusammenfassend für die cis- und trans-Formen II' und II" steht:

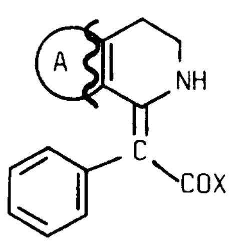

oder

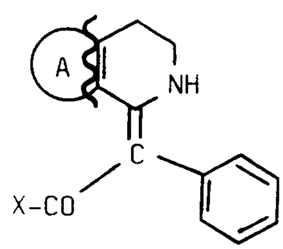

II'　　II''

II

in der X für $OR_1$; $NHR_2$; $NR_3R_4$
und

$R_1$ für Wasserstoff, $C_1$-$C_4$-Alkyl; $C_1$-$C_4$-Alkoxyalkyl

$R_2$ für Wasserstoff; $C_3$-$C_6$-Alkenyl; $C_3$-$C_6$-Alkinyl; $C_3$-$C_6$-Cycloalkyl; $C_3$-$C_6$-Cycloalkenyl; geradkettiges oder verzweigtes $C_1$-$C_6$-Alkyl, welches gewünschtenfalls ein- oder mehrfach mit den nachstehend genannten Substituenten der Gruppen a) bis c), die gleich oder verschieden sein können, substituiert ist:

a) Halogen; Cyano; Hydroxy; Mercapto; $C_1$-$C_4$-Alkoxy; $C_1$-$C_4$-Alkylthio; $C_1$-$C_4$-Alkoxy; $C_1$-$C_4$-Alkylthio; Amino; Mono-$C_1$-$C_4$-Alkylamino; Di-$C_1$-$C_4$-Alkylamino (wobei die Di-$C_1$-$C_4$-Alkylamino (wobei die Alkylradikale gleich oder verschieden sein können), Phenoxy (worin die Phenylgruppe wie unter (b) angegeben substituiert sein kann),

b) Phenyl; gewünschtenfalls ein- oder mehrfach gleich oder Verschieden substituiert durch die Gruppen Halogen, Trifluormethyl, $C_1$-$C_4$-Alkoxy, Hydroxy, Mercapto, $C_1$-$C_4$-Alkylthio, $C_1$-$C_4$-Alkyl, Amino, Mono-$C_1$-$C_4$-Alkylamino, Di-$C_1$-$C_4$-Alkylamino (wobei die Alkylreste gleich oder verschieden sein können), $c_2$-$C_3$-Acylamino, $C_2$-$C_3$-Acyloxy sowie die vicinal an das Phenylsystem gebundene Gruppe -O-$(CH_2)_n$-O (mit n = 1 oder 2)

c) ein 5- oder 6-gliedriger gesättigter oder ganz oder teilweise ungesättigter monocyclischer Heterocyclus mit bis zu 3 Heteroatomen aus der Gruppe N, O, S; sowie als bicyclischer Heterocyclus Indol (wobei die zuvor genannen Heterocyclen ein- oder mehrfach durch $C_1$-$C_4$-Alkyl substituiert sein können,) $C_3$-$C_6$-Cycloalkyl; $C_5$- oder $C_6$-Cycloalkenyl; $C_2$-$C_3$-Acyl; $C_1$-$C_4$-Alkylsulfonyl; oder Phenyl (welches seinerseits bis zu dreifach wie unter b) beschrieben substituiert sein kann);

oder $R_2$ Phenyl ist, das wie oben unter (b) angegeben substituiert sein kann;
$R_3$ und $R_4$ unabhängig voneinander für $C_1$-$C_4$-Alkyl, welches gewünschtenfalls phenylsubstituiert sein kann, wobei der Phenylsubstituent seinerseits wie vorstehend unter b) substituiert sein kann;

oder

$R_3$ und $R_4$ zusammen mit dem Stickstoffatom, an welches sie gebunden sind, für einen ganz- oder teilweise gesättigten heterocyclischen 5- oder 6-Ring (der außerdem noch bis zu 2 weitere Heteroatome aus der Gruppe N, O, S enthalten kann) bilden, wobei der so erhaltene Heterocyclus durch $C_1$-$C_4$-Alkyl, Hydroxy, oder $(CH_2)_p$-$R_5$ (mit p = 0 oder 1) substituiert sein kann

und

$R_5$ für ein Phenylradikal, welches gewünschtenfalls wie vorstehend unter b) substituiert ist, steht;
A steht für die anellierten Ringsysteme

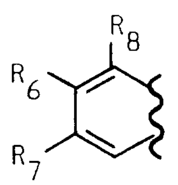
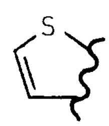
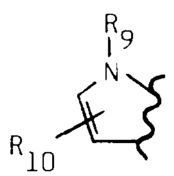
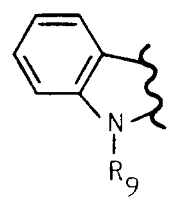

in denen
$R_8$ für Wasserstoff; $C_1$-$C_4$-Alkyl; oder $C_1$-$C_4$-Alkoxy
$R_6$ und $R_7$ die gleich oder verschieden sein können für Wasserstoff; Hydroxy; $C_1$-$C_4$-Alkyl; $C_1$-$C_4$-Alkoxy; Amino; Methansulfonylamino

oder

$R_6$ und $R_7$ gemeinsam für -O-$(CH_2)_n$-O- (mit n = 1 oder 2)
$R_9$ für Wasserstoff; $C_1$-$C_4$-Alkyl
$R_{10}$ für Wasserstoff; oder 2-Phenyl-2-ethoxycarbonylacetyl

steht sowie deren Salze mit physiologisch verträglichen Säuren, Basen oder Komplexbildnern zur Hirnprotektion.

Unter die beschriebene allgemeine Formel I fallen auch die neuen 3,4-Dihydroisochinolinderivate der allgemeinen Formel Ie

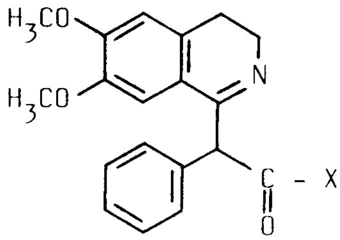

(Ie)

worin X

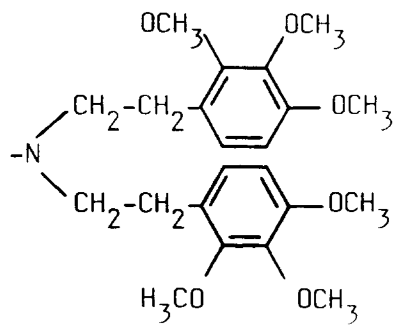

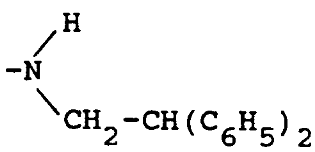

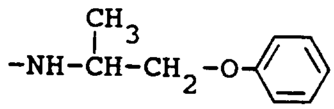

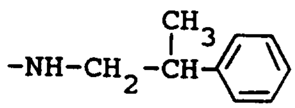

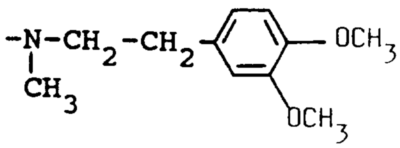

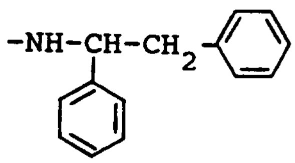

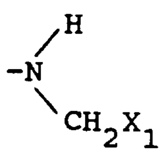

oder

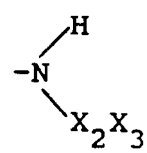

ist,
worin

$X_1$ durch Trifluormethyl oder Ethoxy mono- oder di-substituiertes Phenyl, durch Methoxy und Fluor substituiertes Phenyl oder 2-Methoxy-phenyl ist,

$X_2$ $-CH_2-CH_2-$ oder $-CH_2-CH(CH_3)-$ ist, und

$X_3$ 2,3,4-Trimethoxyphenyl, 2,3-Dimethoxyphenyl, 2,6-Dimethoxyphenyl, 3,6-Dimethoxyphenyl, Thienyl, durch Trifluormethyl oder Ethoxy mono- oder di-substituiertes Phenyl, oder durch Methoxy und Fluor substituiertes Phenyl ist,

oder deren pharmazeutisch annehmbare Salze.

Bevorzugt sind Verbindungen (Ie),
worin X

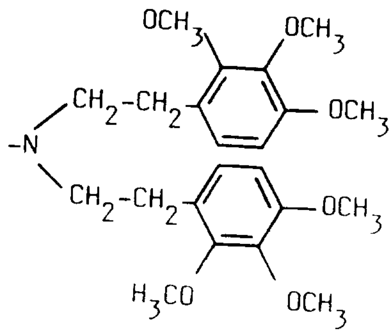

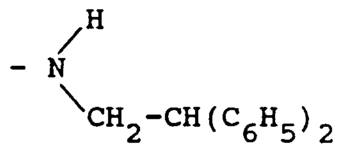

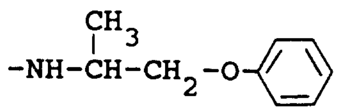

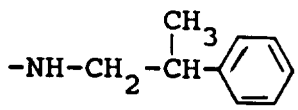

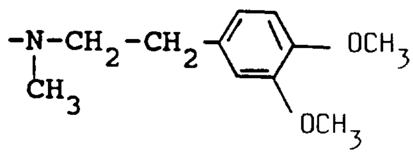

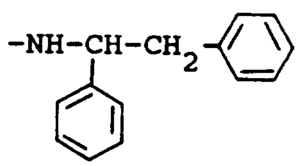

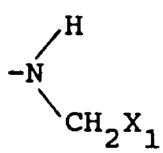

oder

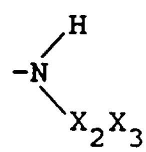

ist,
worin

$X_1$ 2-Methoxyphenyl ist, das zusätzlich durch Fluoro subsituiert sein kann,

$X_2$ $-CH_2-CH_2-$ ist
und

$X_3$ 2,3,4-Trimethoxyphenyl, 2,3-Dimethoxyphenyl, 2,6-Dimethoxyphenyl, 3,6-Dimethoxyphenyl, 2 oder 3-Thienyl, durch Trifluormethyl oder Ethoxy substituiertes Phenyl, oder durch Methoxy und Fluor substituiertes Phenyl ist.

Diese neuen Verbindungen sind auch für die oben beschriebenen Verwendung geeignet.
Bevorzugt ist die Verwendung von carbocyclisch und heterocyclisch anellierten Dihydropyridinen der Formel

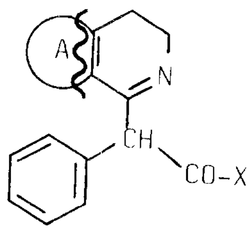

I

sowie deren tautomere Formen der Formel

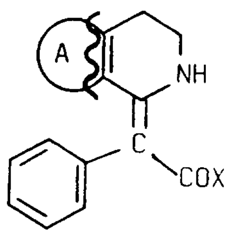

oder

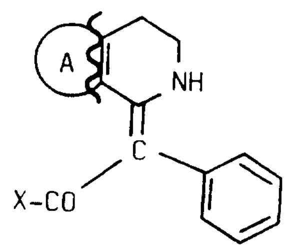

II' II''

II

in der X für $OR_1$ $NHR_2$; $NR_3R_4$

$R_1$ für Methyl oder Ethyl

$R_2$ für Wasserstoff; geradkettiges oder verzweigtes unsubstituiertes $C_1$-$C_5$-Alkyl; Allyl; Propargyl; $C_3$-$C_6$-Cycloalkyl; 3-Chlorphenyl; 2-Methyl-3-chlorphenyl; oder $C_1$-$C_3$-Alkyl, welches einfach mit einem der in nachstehend genannten Substituenten der Gruppen d) bis f) substituiert ist;

d) Cyano, Hydroxy, Methoxy, Dimethylamino

e) Phenyl, 3,4-Methylendioxyphenyl, durch eine, zwei odere 3 Methoxygruppen substituiertes Phenyl, 3-Hydroxy-4-methoxyphenyl,

f) Morpholino, Pyridin-2-yl, Indol-3-yl, Furan-2-yl, Thiophen-2-yl, Pyridin-3-yl, Pyridin-4-yl

$R_3$ und $R_4$ unabhängig voneinander für Methyl; Ethyl; 3-Cyanopropyl; Benzyl; oder 3,4,5-Trimethoxyphenethyl stehen oder

$R_3$ und $R_4$ zusammen mit dem Stickstoffatom an welches sie gebunden sind für Morpholin; Thiomorpholin; Pyrrolidin; Piperazin; 4-Methylpiperazin; 4-Benzylpiperazin; oder 4-(2-Methoxyphenyl)piperazin stehen;

und

A für die anellierten Ringsysteme

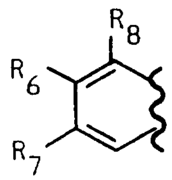

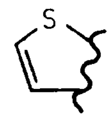

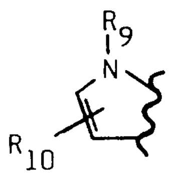

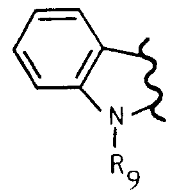

in denen

$R_8$ für Wasserstoff; oder Methoxy
$R_6$ für Methoxy; Hydroxy; Wasserstoff; Amino; oder Methansulfonylamino
$R_7$ für Wasserstoff; Methoxy; oder Hydroxy
$R_9$ für Methyl
$R_{10}$ für 2-Phenyl-2-ethoxycarbonylacetyl; oder Wasserstoff

steht, sowie deren Salze mit physiologisch verträglichen Säuren.

Die Erfindung betrifft somit Dihydroisochinoline der Formel

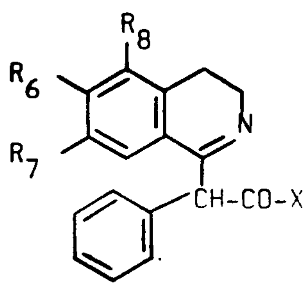

Ia

Dihydro-thieno[3,2-c]pyridine der Formel

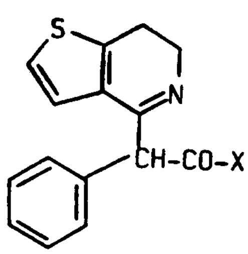

Ib

Dihydro-pyrollo[3,2-c]pyridine der Formel

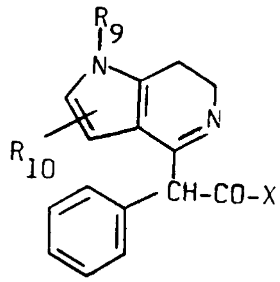

und Dihydro-pyrido[3,4-b]indole der Formel

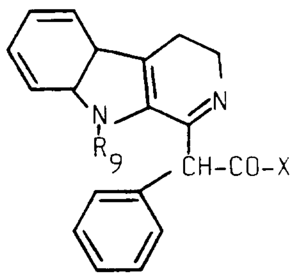

Id

sowie deren tautomere Formen IIa', IIb', IIc' und IId' und die dazu E/Z-isomeren Formen IIa", IIb", IIc" und IId".

Die Verbindungen der Formel Ia - Id sind chiral. Die Erfindung umfaßt auch die beiden R- und S-enantiomeren Formen der Verbindungen der Formel I bei denen die Bedeutung der Reste X, $R_6$, $R_7$, $R_8$, $R_9$ und $R_{10}$ wie zuvor definiert ist.

Die in den nachstehenden Tabellen 1 bis 12 aufgeführten Substanzen liegen jeweils bevorzugt in der tautomeren Form I oder II vor.

Die bevorzugte tautomere Form ist in der Spalte Struktur mit I bzw. II gekennzeichnet.

Namentlich zu nennen sind folgende Verbindungen der Formel I in der tautomeren Form I bzw. II.

In Tabelle 12 sind neue Verbindungen der allgemeinen Formel Ie zusammengefaßt.

Tabelle 1

Strukturtyp:

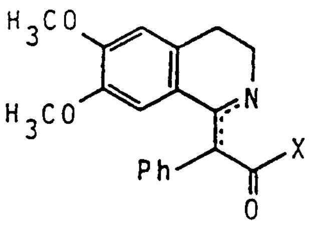

| Nr. | X | Struktur | Salzform |
|---|---|---|---|
| 1. | $OCH_3$ | II | - |
| 2. | $OC_2H_5$ | II | - |
| 3. | $NHCH_3$ | I | Cl |
| 4. | $NHC_2H_5$ | I | - |
| 5. | $NH-(CH_2)_2-CH_3$ | I | Cl |
| 6. | $NH-(CH_2)_3-CH_3$ | I | Cl |
| 7. | $NH-(CH_2)_4-CH_3$ | I | Cl |
| 8. | $NH-CH(CH_3)_2$ | I | Cl |
| 9. | $NH-CH_2-CH(CH_3)_2$ | I | Cl |
| 10. | $NH-(CH_2)_2-CH(CH_3)_2$ | I | Cl |
| 11. | $NH-C(CH_3)_3$ | I | Cl |
| 12. | $NH-CH(CH_3)-C_2H_5$ | I | Cl |
| 13. | $NH-CH_2-CH=CH_2$ | I | Cl |
| 14. | $NH-CH_2-C\equiv CH$ | I | Cl |
| 15. | $NH-(CH_2)_2-OH$ | II | Cl |
| 16. | $NH-CH_2-CH(OH)-CH_3$ | I | Cl |
| 17. | $NH-(CH_2)_2-OCH_3$ | II | Cl |
| 18. | $NH-(CH_2)_3-OCH_3$ | II | Cl |
| 19. | $NH-(CH_2)_2-N(CH_3)_2$ | I | $Cl_2$ |
| 20. | $NH-(CH_2)_3-N(CH_3)_2$ | II | $Cl_2$ |
| 21. | $NH-(CH_2)_2-$ 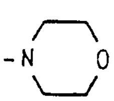 | II | - |

| Nr. | X | Struktur | Salzform |
|---|---|---|---|
| 22. | 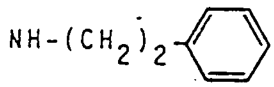 | I | Cl |
| 23. | 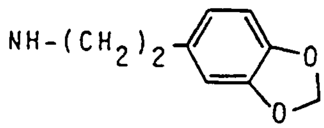 | I | - |
| 24. | 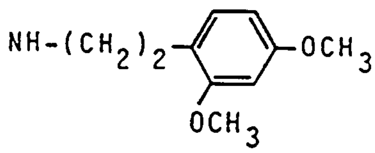 | I | - |
| 25. | 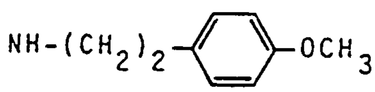 | I | - |
| 26. | 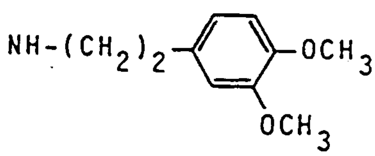 | II | - |
| 27. | 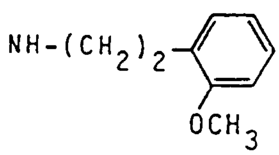 | II | Cl |
| 28. | 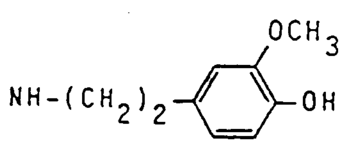 | I | - |
| 29. | 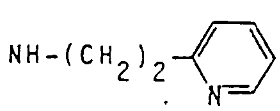 | I | - |

| Nr. | X | Struktur | Salzform |
|---|---|---|---|
| 30. | NH-(CH$_2$)$_2$- 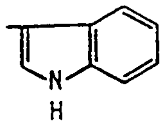 | II | - |
| 31. | NH- 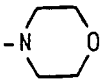 | I | - |
| 32. | NH-CH$_2$- 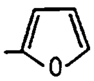 | II | - |
| 33. | NH- 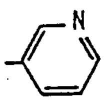 | I | Cl |
| 34. | NH- 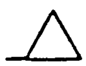 | I | Cl |
| 35. | NH- 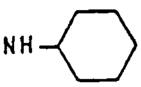 | II | - |
| 36. | N(CH$_3$)$_2$ | I | Cl |
| 37. | N(C$_2$H$_5$)$_2$ | I | Cl |
| 38. | N(CH$_2$-CH$_2$- 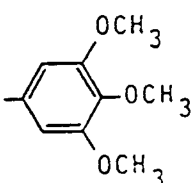 )$_2$ | I | Cl |
| 38a. | N(CH$_2$-CH$_2$- 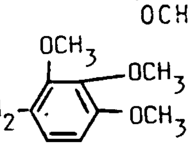 )$_2$ | I | Cl |
| 39. | 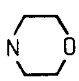 | I | Cl |

| Nr. | X | Struktur | Salzform |
|---|---|---|---|
| 40 | 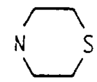 | I | - |
| 41. | 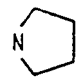 | I<br>II | - |
| 42. | 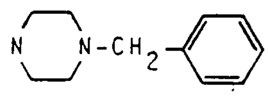 | II | $Cl_2$ |
| 43 | 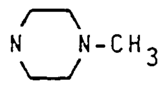 | I | - |
| 44 | 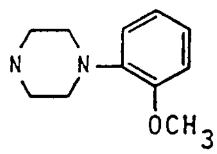 | I | Cl |

Tabelle 2

Strukturtyp:

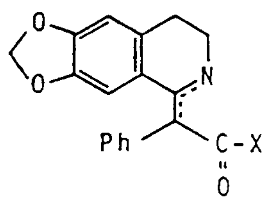

| Nr. | X | Struktur | Salzform |
|---|---|---|---|
| 45. | $OC_2H_5$ | II | - |
| 46. | NH-$(CH_2)_2$- 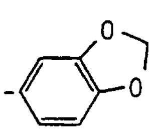 | II | Cl |
| 47. | NH-$(CH_2)_2$ 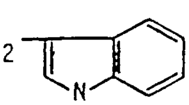 | II | - |

| Nr. | X | Struktur | Salzform |
|---|---|---|---|
| 48. | NH-$(CH_2)_2$-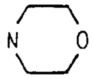 | II | - |
| 49. | 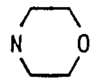 | II | - |
| 50. | 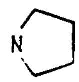 | I | - |

Tabelle 3

Strukturtyp:

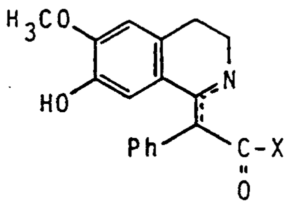

| Nr. | X | Struktur | Salzform |
|---|---|---|---|
| 51. | $OC_2H_5$ | I<br>II | - |
| 52. | NH-$(CH_2)_3$-$CH_3$ | I | - |
| 53. | NH-$(CH_2)_4$-$CH_3$ | I | - |
| 54. | NH-CH$(CH_3)_2$ | I | Cl |
| 55. | NH-$CH_2$-CH$(CH_3)_2$ | I | - |
| 56. | NH-$CH_2$-CH=$CH_2$ | I | - |
| 57. | NH-$(CH_2)_2$-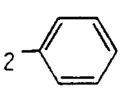 | I | Cl |
| 58. | NH-$(CH_2)_2$-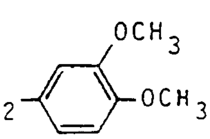 | I | Cl |

| Nr. | X | Struktur | Salzform |
|---|---|---|---|
| 59. | 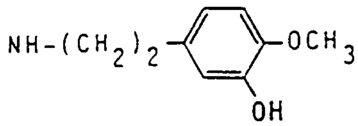 | I | |
| 60. | 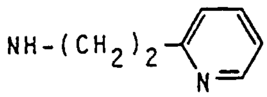 | I | |
| 61. | 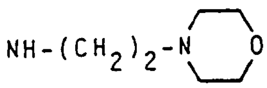 | I | - |
| 62. | 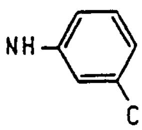 | I | - |
| 63. | 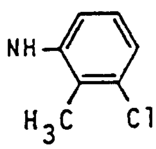 | I | Cl |
| 64. | 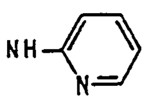 | I | - |
| 65. | 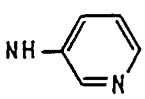 | I | Cl |
| 66. | 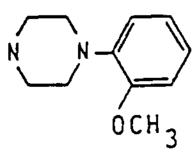 | I | Cl |
| 67. | 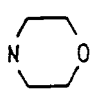 | I | Cl |
| 68. | 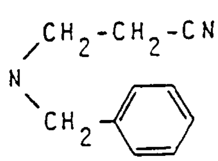 | I | Cl |

Tabelle 4

Strukturtyp:

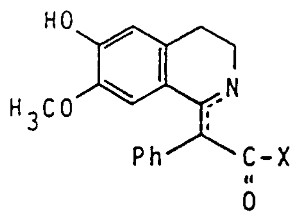

| Nr. | X | Struktur | Salzform |
|---|---|---|---|
| 69. | $N(C_2H_5)_2$ | I | |

Tabelle 5

Strukturtyp:

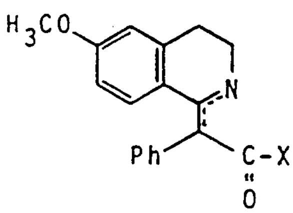

| Nr. | X | Struktur | Salzform |
|---|---|---|---|
| 70. | $NHCH_3$ | I | |
| 71. | $NHC_2H_5$ | I | |
| 72. | $NH-CH_2-$ 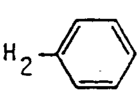 | I | |
| 73. | $NH(CH_2)_2-$ 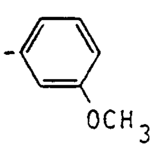 | I | |
| 74. | $N(CH_3)C_2H_5$ | I | |

Tabelle 6

Strukturtyp:

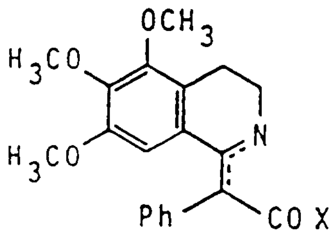

| Nr. | X | Struktur |
|---|---|---|
| 75. | 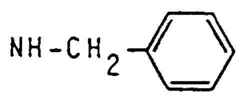 | I |
| 76. | 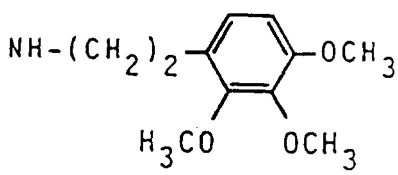 | I |
| 77. | $N(CH_3)C_2H_5$ | I |
| 78. | 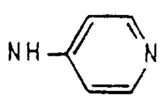 | II |
| 79. | 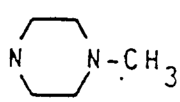 | I |

Tabelle 7

Strukturtyp:

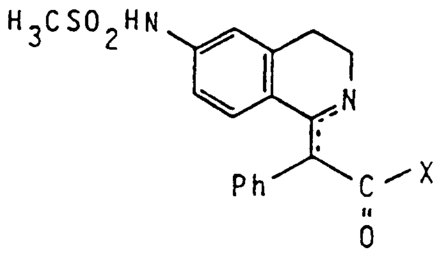

| Nr. | X | Struktur | Salzform |
|---|---|---|---|
| 80 | $OC_2H_5$ | II | - |

Tabelle 8

Strukturtyp:

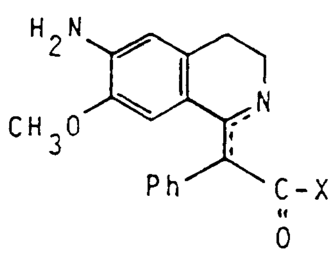

| Nr. | X | Struktur | Salzform |
|---|---|---|---|
| 81. | $OC_2H_5$ | II | Cl |

Tabelle 9

Strukturtyp:

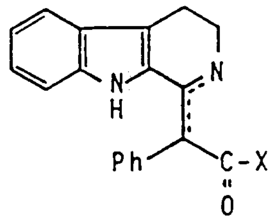

| Nr. | X | Struktur | Salzform |
|---|---|---|---|
| 82. | $OC_2H_5$ | I<br>II | - |
| 83. | $NH-(CH_2)_2-$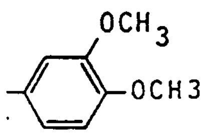 | II | - |
| 84. | $NH-(CH_2)_2-$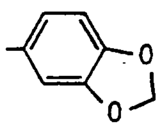 | II | - |
| 85. | $NH-(CH_2)_2-$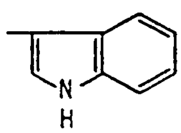 | I<br>II | - |
| 86. | 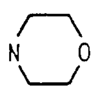 | I | - |
| 87. | $NH-CH_2-CH(CH_3)_2$ | II | - |
| 88. | $NH-(CH_2)_3-N(CH_3)_2$ | II | - |
| 89. | $NH-(CH_2)_2-$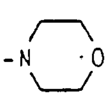 | II | - |

Tabelle 10

Strukturtyp:

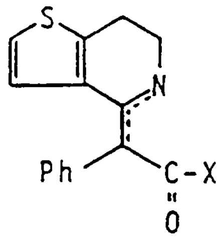

| Nr. | X | Struktur | Salzform |
|---|---|---|---|
| 90. | $OC_2H_5$ | | |
| 91. | $NH-(CH_2)_2-$ 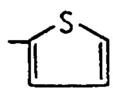 | II | - |

Tabelle 11

Strukturtyp:

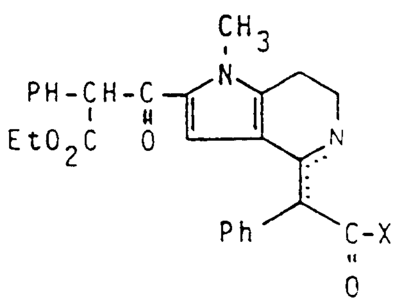

| Nr. | X | Struktur | Salzform |
|---|---|---|---|
| 92. | $OC_2H_5$ | II | - |

Tabelle 12

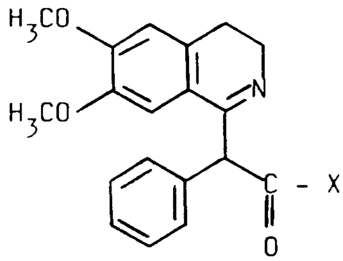

Strukturtyp I

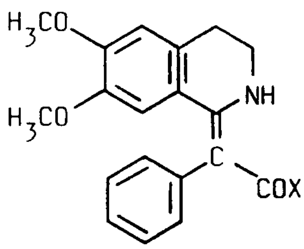

oder

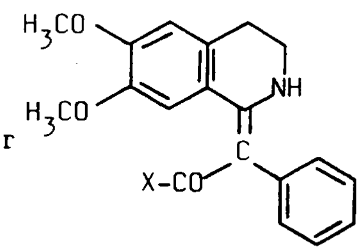

II' II''

Strukturtyp II

| Verbindung | X | Strukturtyp | Fp[°C] |
|---|---|---|---|
| A | 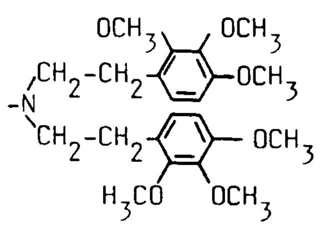 .HCl | I | 56-64 |
| B | 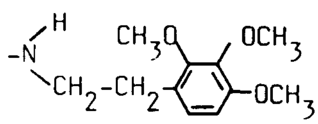 .HCl | I | 176-184 |

| Verbindung | (Salzform) | X | Strukturtyp | Fp[°C] |
|---|---|---|---|---|
| C | | 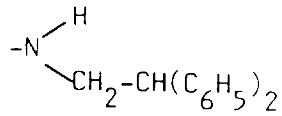 | I | 166-168 |
| D | | 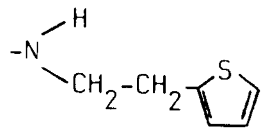 | II | 102-104 |
| E | (Cl) | 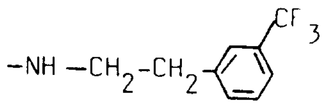 | I | 187 |
| F | (-) | 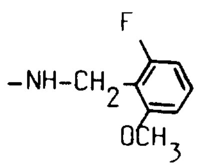 | I | 94-96 |
| G | (Cl) | 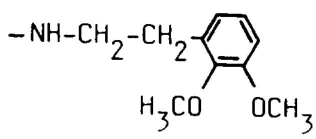 | II | 139-142 |
| H | (-) | 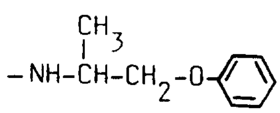 | II | 133-135 |
| J | (-) | 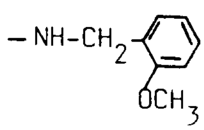 | II | 143-145 |
| K | (-) | 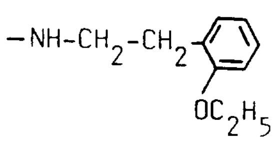 | II | 96-98 |

| Verbindung (Salzform) | X | Strukturtyp | Fp[°C] |
|---|---|---|---|
| L (-) | 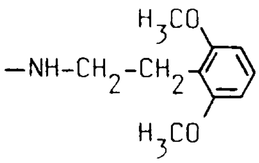 | II | 118-120 |
| M (-) | 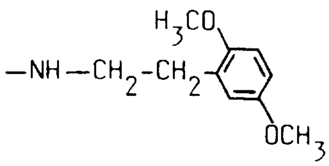 | II | 112-114 |
| N (Cl) | 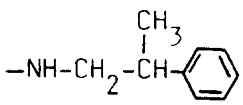 | I | 95-99 |
| O (-) | 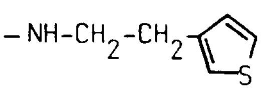 | I | 114-116 |
| P (-) | 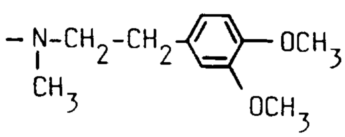 | I | 66-73 |
| Q (Cl) | 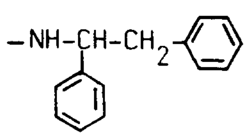 | II | 205-209 |

In den im Text verwendeten Definitionen können die Reste und Gruppen jeweils gleich oder verschieden sein, d.h. wenn einer der zuvor genannten Substituenten in einem bestimmten Molekül mehrfach vorkommt, kann die jeweilige Bedeutung im Rahmen der Definitionsbreite frei gewählt werden.

Mit Alkyl sind $C_1$-$C_6$-Alkyl- und $C_1$-$C_4$-Alkylradikale gemeint die ihrerseits substituiert sein können oder als Alkylradikale Bestandteil einer funktionellen Gruppe - wie Alkoxy oder Alkylthio - sind. Die Alkylradikale umfassen die Methyl-, Ethyl-, n-Propyl-, iso-Propyl, n-Butyl-, sek-Butyl-, iso-Butyl-, tert-Butylradikale sowie die verschiedenen isomeren Pentyl- und Hexylradikale, wie etwa das Isopentyl, das Neopentyl, das n-Pentyl und das n-Hexylradikal.

Die vorstehende Definition gilt somit auch wenn das Alkylradikal seinerseits substituiert und/oder selbst Bestandteil einer Alkoxyalkyl-, Alkoxycarbonyl-, Alkoxy-, Alkylthio-, Alkylsulfonyl-, Monoalkylamino-, Alkylmethyl-, Alkylthiomethyl-, Dialkylaminogruppe ist oder das Alkylradikal als Substituent an ein aromatisches heterocyclisches oder carboxyclisches System gebunden ist.

Halogene sind Fluor, Chlor, Brom und Jod, vorzugsweise Fluor, Chlor und Brom und in zweiter Linie Jod.

$C_3$-$C_6$-Cycloalkyl sind Cyclopropan, Cyclobutan, Cyclopentan und Cyclohexan. $C_5$-$C_6$-Cycloalkene sind z.B. Cyclopenten, Cyclohexen und Cyclohexadien.

Das $C_2$- und $C_3$-Acylradikal steht für das Acetyl und das Propionylradikal.

$C_3$-$C_6$-Alkine sind die isomeren Hexine, Pentine, Butine und Propine bevorzugt ist Propargyl

$C_3$-$C_6$-Alkene sind die isomeren Hexene, Pentene, Butene und Propene bevorzugt ist Allyl

Als ungesättigte Heterocyclen sind unter anderem zu nennen:
Furan, Pyran, Pyrrol, Pyrazol, Imidazol, Pyridin, Pyrazin, Pyrimidin, Pyridazin, Thiophen, Thiazol, Oxazol, 1,2,4-Triazol, 1,2,3-Triazol, 1,2,4-Triazin, 1,3,5-Triazin, Indol.

Als 5- oder 6-gliedrige ganz oder teilweise gesättigte monocyclische Heterocyclen sind unter anderem zu nennen:

Imidazolidin, Pyrazolidin, Pyrrolidin, Piperidin, Piperazin, Morpholin, Thiomorpholin, Tetrahydrofuran, Tetrahydrothiophen, 1,4-Dioxin, Imidazolin, Pyrazolin, Pyrrolin, etc.

Die Verbindungen der Formel I bzw. II sind Basen und können auf übliche Weise mit anorganischen oder organischen Säuren sowie Salz- und Komplexbildnern in beliebige physiologisch unbedenkliche Addukte (Salze) überführt werden.

Zur Salzbildung geeignete Säuren sind beispielsweise Salzsäure, Bromwasserstoffsäure, Jodwasserstoffsäure, Fluorwasserstoffsäure, Schwefelsäure, Phosphorsäure, Salpetersäure, Essigsäure, Propionsäure, Buttersäure, Capronsäure, Valeriansäure, Oxalsäure, Malonsäure, Bernsteinsäure, Maleinsäure, Fumarsäure, Milchsäure, Weinsäure, Zitronensäure, Apfelsäure, Benzoesäure, p-Hydroxybenzoesäure, Phthalsäure, Zimtsäure, Salicylsäure, Ascorbinsäure, Methansulfonsäure und dergleichen.

Die meisten Verbindungen der allgemeinen Formel I und deren Herstellungsmethoden sind aus den eingangs genannten EP-A 37 934 und EP-A 251 194 bekannt. In diesen Veröffentlichungen sind die Verbindung der Formel Ie nicht genannt.

Wie bereits eingangs erwähnt ist der Gegenstand der vorliegenden Erfindung die Verwendung der aus der genannten EP-A 251 194 bekannten Verbindungen und insbesondere der neuen Verbindung des Beispiels 38A als hirnprotektive Mittel. Die Verbindungen sind zur Behandlung von degenerativen und nekrotischen Erkrankungen des Gehirns vorteilhaft. Ebenso ist die vorbeugende Behandlung von durch solche Krankheiten gefährdeten Patienten möglich. Wie durch die weiter unten beschriebenen Versuche gezeigt wird, beruht die Wirkung der Verbindungen nicht auf einer Verbesserung der Gewebsdruchblutung. Die Verbindungen sind somit für eine neuartige Behandlung von Epilepsie und der Alzheimer-Krankheit geeignet, und insbesondere bei der Behandlung von Patienten, die einen Schlaganfall erlitten haben oder gefährdet sind, einen Schlaganfall zu erleiden.

Die folgenden Untersuchungsergebnisse zeigen die überraschende Wirkung der Verbindungen. Die Untersuchungen wurden insbesondere mit Verbindung A (Beispiel 38A)

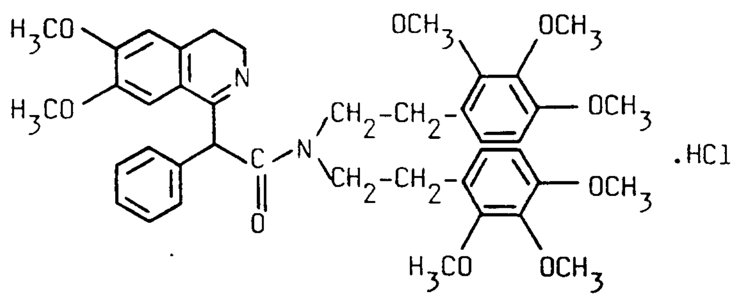

als Vertreterin der Verbindungen der allgemeinen Formel I ausgeführt.

Ischämietoleranz am Gerbil:
(z.B. Suzuki, R. et al., Acta Neuropath (Berl.) 1983, 60:207-216, 217-222; Yoshidomi, M. et al., J. Neurochem. 1989, 53:1589-1594)
Ischämie durch Occlusion der Arteria carotis für 10 Minuten unter Etheranaesthesie. Die Verbindung A wurde mit 1 und 10 mg/kg s.c. 4 x innerhalb von 24 Stunden, beginnend 2 Stunden nach Wiederöffnung der Arterien, appliziert.

72 Stunden nach Gefäßocclusion wurden die Tiere getötet und die Gehirne zur histologischen Untersuchung präpariert. Als Nachweis eines ischämischen Schadens beziehungsweise dessen Reduzierung wurde im Bereich der $CA_1$-Region des Hippocampus in einem definierten Ausschnitt des histologischen Präparates die Schädigung der Zellen beurteilt. Die Untersuchung wurde an Gruppen von je 5 Tieren ausgeführt.

In der Gruppe, in der den Tieren keine zu untersuchende Verbindung verabreicht wurde, zeigten alle Tiere eine deutliche Schädigung der untersuchten $CA_1$-Region. Im Gegensatz dazu zeigte sich bei Verabreichung der Verbindung

A ein deutlicher dosisabhängiger Schutz gegen den Hypoxieschaden. Die Verabreichung von 1 mg/kg der Verbindung A zeigen nur 2 von 5 Tieren Schädigungen der $CA_1$-Region. Bei Verabreichung von 10 mg/kg der Verbindung A zeigt keines der 5 Tiere eine Schädigung.

Aus diesem Ergebnis kann geschlossen werden, daß die Verbindungen der allgemeinen Formel I zur kurativen Behandlung von neurologischen Schäden, z.B. verursacht durch Schlaganfall, verwendet werden können.

Das Ergebnis dieser Untersuchung zeigt auch, daß die Verbindungen die Blut-Hirn-Schranke überwinden, was ein wesentliches Merkmal der Erfindung ist.

Die folgenden Untersuchungen an isolierten Zellkulturen zeigen die Wirkung der untersuchten Verbindungen. Ferner kann man aus diesen Untersuchungsergebnissen ebenfalls (wie aus den Untersuchungsergebnissen am Gerbil) schließen, daß die Verbindungen der allgemeinen Formel I für die in dieser Patentanmeldung genannten Behandlungen verwendet werden können.

In isolierten Zellkulturen (z.B. neutrophile Granulozyten, HL 60-Zellen, Thrombozyten, u.a.) konnte Verbindung A den durch unterschiedliche Agonisten (z.B. PAF, Leukotriene, Endothelin, FMLP oder Isoprenalin) provozierten "Calciumoverload" und Zelltod dosisabhängig inhibieren ($IC_{50}$-Werte im Bereich von 1 μM).

Gemessen wurden die halbmaximalen Hemmkonzentrationen der Testsubstanzen, die den FMLP (1 nM)-induzierten Calciumtransienten an $FLUO_3$-beladenen HL60-Zellen inhibieren.

| Verbindung Nr. | halbmaximale Hemmkonzentration (Durchschnittswert $10^6$ Zellen in Suspension) |
|---|---|
| 43 | $5.10^{-5}$M |
| 47 | $4,8.10^{-5}$M |
| 38a (Verbindung A) | $5,4.10^{-6}$M |

Diese oben erwähnten Untersuchungsmethoden sind in W.K. Pollock, T.J. Rink, R.F. Irvine, Biochem. J. 235:869-877 (1986) beziehungsweise J.E. Merritt, R. Jacob, T.J. Hallam, J. of Biol. Chem. 264:1522-1527 (1989) beschrieben.

An einzelnen HL-60 Zellen wurde mittels patch clamp Technik elektrophysiologisch der transmembranäre Einstrom durch "Unselective Cation Channels" nach ATP Stimulation gemessen. Dieser Einstrom wird durch Verbindung A inhibiert ($IC_{50}$-Wert: 7 nM).

Diese Befunde belegen den direkten Angriffspunkt an isolierten Zellen. Im lebenden Organismus wandern speziell die in weiteren Untersuchungen verwendeten Granulozyten bzw. Leukozyten nach einer Schädigung von Gehirnzellen (z.B. durch Schlaganfall) in das geschädigte Areal ein, wo sie durch Calcium vermittelte Prozesse aktiviert werden, zerfallen und dabei gewebeschädigende, darunter auch chemotaktische Mediatoren freisetzen (z.B. PAF, Leukotriene, Prostaglandine, FMLP u.a.). Durch Chemotaxis angelockte zusätzliche Leukozyten vergrößern das geschädigte Areal. Die oben beschriebenen Untersuchungsergebnisse zeigen, daß dieser Circulus vitiosus durch Verabreichung der oben beschriebenen aktiven Substanzen blockiert werden kann. Dadurch bleibt der neurologische Schaden begrenzt.

Es konnte gezeigt werden, daß klassische Calciumantagonisten (z.B. Verapamil, Nifedipine, Diltiazem) die Leukozytenaktivierung nicht inhibieren.

Weitere Untersuchungen mit Verbindung A unterstützen die obige Feststellung:

An isolierten corticalen und hippocampalen Neuronenzellen aus fetalen Rattenhirnen (Präparation nach H.W. Müller und W. Seifert, Proc. Natl. Acad. Sci. USA 81: 1248-1252, 1984; J. Neurosci. Res. 8: 195-204, 1982; sowie Müller and Seifert in "Methods for Serum Free Culture of Neuronal and Lymphoid Cells," p. 67-77, A.R. Liss Inc., 150 Fifth Ave., New York, N.Y. 10011, 1984) wurde der direkte neuronale Angriff der Verbindung A nachgewiesen. In $FURA_2$-beladenen Einzelzellen wurden die Konzentrations-Zeit-Kurven (Calciumtransient) des cytoplasmatischen $Ca^{2+}$ registriert (Methode: modifiziert nach J.A. Connor, Proc. Natl. Acad. Sci. 83: 6179-6183, 1986). Sowohl nach mechanischer Läsion als auch nach Gabe exzitatorischer Aminosäuren (E.A.A., Glutamat, Kainat, Quisqualat und NMDA) wurde ein starker Anstieg der cytoplasmatischen Calciumkonzentrationen provoziert, der in allen Fällen dosisabhängig ($IC_{50}$ bei 3 μM) durch Verbindung A inhibierbar war.

Der Wirkungsmechanismus dieser Hemmung wurde sowohl an neuronalen Zellkulturen als auch an menschlichen neutrophilen Granulozyten und HL 60-Zellen und Thrombozyten untersucht. Es konnte gezeigt werden, daß Verbindung A den transmembranären Calciumeinstrom in die Zellen hemmt, der durch Rezeptoragonisten (z.B. EAA, FMLP, Leukotriene, PAF, Endothelin u.a.) stimuliert wird. Dieser von T.J. Hallam and T.J. Rink (Tips 10: 8-10, 1989) als

"receptor mediated $Ca^{2+}$ entry (RMCE)" bezeichnete Influx ist durch klassische Calciumantagonisten nicht inhibierbar. Klassische Calciumantagonisten können die Leukozyten und Thrombozyten-Aktivierung nicht verhindern, da diese Zellen keine spannungsabhängigen $Ca^{2+}$-Kanäle haben. Die Blockade des transmembranären Calciumeinstroms konnte elektrophysiologisch (Voltage clamp-Technik) an HL6O-Zellen und Neuronenzellen bestätigt werden.

Die Versuchsergebnisse werden in der folgenden Tabelle zusammengefaßt. Die Verbindungen sind in den Tabellen 1-12 angegeben.
%H = % Hemmung des transmembranären $Ca^{2+}$-Einstroms (Neuronenzellen)

| Verbindung Nr. | % H |
|---|---|
| 2 | 83.78 |
| 6 | 72.79 F |
| 7 | 79.94 F |
| 10 | 67.83 F |
| 16 | 74.58 F |
| 22 | 87.95 F |
| 23 | 72.67 |
| 24 | 91.97 |
| 25 | 82.93 |
| 26 | 46.05 |
| 27 | 93.80 |
| 30 | 78.62 |
| 32 | 61.53 |
| 37 | 65.34 F |
| 42 | 58.78 |
| 45 | 62.72 |
| 46 | 82.72 |
| 47 | 52.29 |
| 57 | 54.19 |
| 83 | 76.84 |
| 87 | 61.91 |
| 89 | 53.29 |
| 91 | 66.76 |
| 92 | 42.12 |

| Verbindung Nr. | % H |
|---|---|
| A | 88.08 |
| B | 64.15 |
| C | 86.93 |

(fortgesetzt)

| Verbindung Nr. | % H |
|---|---|
| D | 69.41 |
| E | 93.19 |
| F | 76.64 |
| G | 67.72 |
| H | 79.69 |
| J | 83.81 |
| K | 98.89 |
| L | 99.38 |
| M | 73.21 |
| N | 98.27 |
| O | 90.34 |
| P | 74.58 |
| Q | 81.86 |

Aus diesen Versuchsergebnissen kann geschlossen werden, daß für die Herstellung hirnprotektiver Mittel die Verwendung von Verbindungen der allgemeinen Formel I vorteilhaft ist, worin A

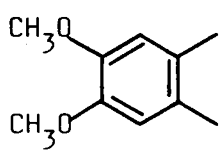

oder

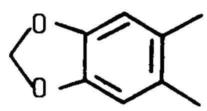

ist,

X $OC_2H_5$, oder
$NHR_1$, worin $R_1$ Alkyl mit 4 oder 5 Kohlenstoffatomen,

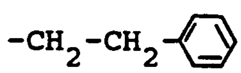

(worin die Phenylgruppe durch eine, zwei oder drei Methoxygruppen, $-O-CH_2-O-$, $CF_3$, F, $OC_2H_5$ oder

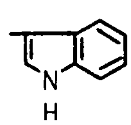

substituiert sein kann) oder $R_1$ $-CH_2-CH(C_6H_5)_2$ ist,
oder X $NR_3R_4$ ist, insbesondere worin
X $N(C_2H_5)_2$ oder

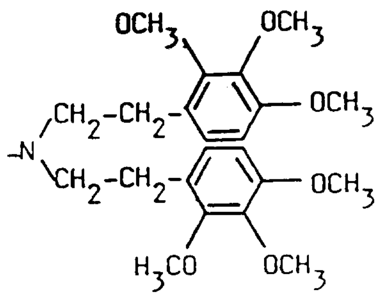

ist.

Die Verbindungen können oral, parenteral oder topisch verabreicht werden. Geeignete Anwendungsformen sind beispielsweise Tabletten, Kapseln, Zäpfchen, Lösungen, Säfte, Emulsionen, Aerosole oder dispersible Pulver. Entsprechende Tabletten können beispielsweise durch Mischen des oder der Wirkstoffe mit bekannten Hilfsstoffen, beispielsweise inerten Verdünnungsmitteln, wie Calciumcarbonat, Calciumphosphat oder Milchzucker, Sprengmitteln, wie Maisstärke oder Alginsäure, Bindemitteln, wie Stärke oder Gelatine, Schmiermitteln, wie Magnesiumstearat oder Talk, und/oder Mitteln zur Erzielung des Depoteffektes, wie Carboxypolymethylen, Carboxymethylcellulose, Celluloseacetatphthalat, oder Polyvinylacetat erhalten werden. Die Tabletten können auch aus mehreren Schichten bestehen.

Entsprechend können Dragees durch Überziehen von analog den Tabletten hergestellten Kernen mit üblicherweise in Drageehüllen verwendeten Mitteln, beispielsweise Kollidon oder Schellack, Gummi arabicum, Talk, Titandioxid oder Zucker, hergestellt werden. Zur Erzielung eines Depoteffektes oder zur Vermeidung von Inkompatibilitäten kann der Kern auch aus mehreren Schichten bestehen. Desgleichen kann auch die Drageehülle zur Erzielung eines Depoteffektes aus mehreren Schichten bestehen, wobei die oben bei den Tabletten erwähnten Hilfsstoffe verwendet werden können.

Säfte der erfindungsgemäßen Wirkstoffe bzw. Wirkstoffkombinationen können zusätzlich noch ein Süßungsmittel, wie Saccharin, Cyclamat, Glycerin oder Zucker sowie ein geschmacksverbesserndes Mittel, z.B. Aromastoffe, wie Vanillin oder Orangenextrakt, enthalten. Sie können außerdem Suspendierhilfsstoffe oder Dickungsmittel, wie Natriumcarboxymethylcellulose, Netzmittel, beispielweise Kondensationsprodukte von Fettalkoholen mit Ethylenoxid, oder Schutzstoffe, wie p-Hydroxybenzoate, enthalten.

Injektionslösungen werden in üblicher Weise, z.B. unter Zusatz von Konservierungsmitteln, wie p-Hydroxybenzoaten, oder Stabilisatoren, wie Alkalisalzen der Ethylendiamintetraessigsäure, hergestellt und in Injektionsflaschen oder Ampullen abgefüllt.

Die eine oder mehrere Wirkstoffe bzw. Wirkstoffkombinationen enthaltenden Kapseln können beispielsweise hergestellt werden, indem man die Wirkstoffe mit inerten Trägern, wie Milchzucker oder Sorbit, mischt und in Gelatinekapseln einkapselt.

Geeignete Zäpfchen lassen sich beispielsweise durch Vermischen mit dafür vorgesehenen Trägermitteln, wie Neutralfetten oder Polyethylenglykol bzw. dessen Derivaten herstellen.

Die Verbindungen können erfindungsgemäß enteral, parenteral oder topisch verabreicht werden, vorteilhaft in einer Menge von 0,05 bis 500 mg pro Dosis für erwachsene Menschen. Vorzugsweise werden bei oraler Verabreichung 0,1 bis 500 mg pro Dosis, und bei i.v. Verabreichung 0,05 bis 150 mg pro Dosis eingesetzt.

Formulierungsbeispiele

Beispiel 1: Tabletten

| | |
|---|---|
| Wirkstoff gemäß Erfindung | 40,0 mg |
| Milchzucker | 100,0 mg |

(fortgesetzt)

| | |
|---|---|
| Maisstärke | 50,0 mg |
| kolloidale Kieselsäure | 2,0 mg |
| Magnesiumstearat | 3,0 mg |
| insgesamt | 200,0 mg |

Herstellung:

Der Wirkstoff wird mit einem Teil der Hilfsstoffe gemischt und mit einer Lösung der löslichen Stärke in Wasser granuliert. Nach dem Trocknen des Granulats wird der Rest der Hilfsstoffe zugemischt und die Mischung zu Tabletten verpreßt.

Beispiel 2: Dragées

| | |
|---|---|
| Wirkstoff gemäß Erfindung | 20,0 mg |
| Milchzucker | 100,0 mg |
| Maisstärke | 65,0 mg |
| kolloidale Kieselsäure | 2,0 mg |
| lösliche Stärke | 5,0 mg |
| Megnesiumstearat | 3,0 mg |
| insgesamt | 195,0 mg |

Herstellung:

Der Wirkstoff und die Hilfsstoffe werden, wie in Beispiel 1 beschrieben, zu Tablettenkernen verpreßt, die mit Zucker, Talcum und Gummi arabicum in üblicher Weise dragiert werden.

Beispiel 3: Suppositorien

| | |
|---|---|
| Wirkstoff gemäß Erfindung | 50,0 mg |
| Milchzucker | 250,0 mg |
| Suppositorienmasse q.s. ad | 1,7 g |

Herstellung:

Der Wirkstoff und der Milchzucker werden miteinander vermischt und die Mischung in der geschmolzenen Suppositorienmasse gleichmäßig suspendiert. Die Suspensionen werden in gekühlte Formen zu Suppositorien von 1,7 g Gewicht ausgegossen.

Beispiel 4: Ampullen

| | |
|---|---|
| Wirkstoff gemäß Erfindung | 20,0 mg |
| Natriumchlorid | 5,0 mg |
| Bi-destilliertes Wasser q.s. ad | 2,0 ml |

Herstellung:

Der Wirkstoff und das Natriumchlorid werden in bi-destilliertem Wasser gelöst und die Lösung in Ampullen steril abgefüllt.

<u>Beispiel 5: Ampullen</u>

| | |
|---|---|
| Wirkstoff gemäß Erfindung | 10,0 mg |
| Natriumchlorid | 7,0 mg |
| Bi-destilliertes Wasser q.s. ad | 1,0 mg |

<u>Beispiel 6: Tropfen</u>

| | |
|---|---|
| Wirkstoff gemäß Erfindung | 0,70 g |
| p-Hydroxybenzoesäuremethylester | 0,07 g |
| p-Hydroxybenzoesäurepropylester | 0,03 g |
| Entmineralisiertes Wasser q.s. ad | 100,00 ml |

Herstellung:

Der Wirkstoff und die Konservierungsmittel werden in entmineralsiertem Wasser gelöst, die Lösung filtriert und in Flaschen zu je 100 ml abgefüllt.

Wie bereits oben erwähnt worden ist, sind die Verbindungen der allgemeinen Formel Ie neu. Sie können analog zu den in EP-A-00 37 934 und EP-A-251 194 beschriebenen Verfahren hergestellt werden:

a) durch Cyclisierung eines Phenylmalonsäurediamids der Formel III

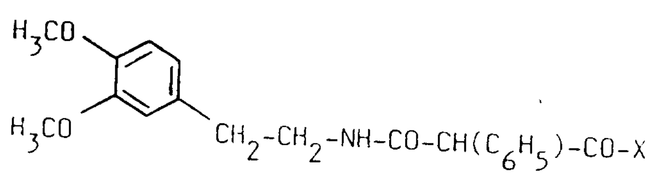

III

in Gegenwart eines Kondensationsmittels

oder

b) ausgehend von Carbonsäuren oder Carbonsäurehalogeniden der Formel IV, in der Y für OH oder Halogen steht, durch Umsetzung mit dem Amin der allgemeinen Formel VI

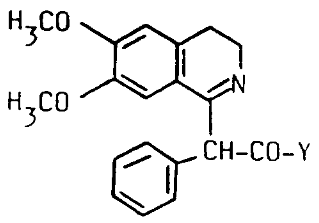

+ XH

IV

VI

in Gegenwart eines geeigneten Kondensationsmittels.

In den obigen Formeln III und IV ist die Gruppe X wie für Formel Ie definiert.

Als Kondensationsmittel für das erfindungsgemäße Verfahren a) eignen sich zahlreiche Lewissäuren, wie z.B. Phosphoroxichlorid, Bortrifluorid, Zinntetrachlorid oder Titantetrachlorid, aber auch starke Mineralsäuren wie Schwefelsäure, Fluorsulfonsäuren, Fluorwasserstoffsäure oder Polyphosphorsäure. Sie werden gewöhnlich im Überschuß eingesetzt. Bevorzugt wird Phosphoroxichlorid.

Die Cyclisierungsreaktion kann in Gegenwart oder in Abwesenheit eines Lösungsmittels durchgeführt werden. Geeignet sind alle inerten Lösungsmittel, soweit sie eine ausreichende Löslichkeit für die Reaktionspartner besitzen und einen ausreichend hohen Siedepunkt aufweisen. Beispiele sind Benzol, Alkylbenzole, Chlorbenzole, Dekalin, Chloroform, Methylenchlorid, Acetonitril und dergl. Eine Variante des Verfahrens besteht darin, das Kondensationsmittel, beispielsweise Phosphoroxichlorid, selbst als Lösungsmittel zu verwenden.

Bezüglich der Reaktionstemperatur bestehen keine speziellen Bedingungen. Die erfindungsgemäße Umsetzung kann innerhalb eines großen Temperaturbereiches, vorzugsweise unter Erwärmen oder Erhitzen bis etwa zum Siedepunkt des Lösungsmittels durchgeführt werden.

Die Amidierungsreaktion b) kann prinzipiell unter den gleichen Bedingungen wie Reaktion a) durchgeführt werden. Als Kondensationsmittel sind zusätzlich Carbodiimide - wie Cyclohexylcarbodiimid - oder Carbonyldiimidazol zu nennen.

Beispiel

3,4-Dihydro-1-benzyl-6,7-dimethoxy-α-[di-2-(2,3,4-trimethoxyphenyl)ethyl]aminocarbonyl-isochinolin-hydrochlorid

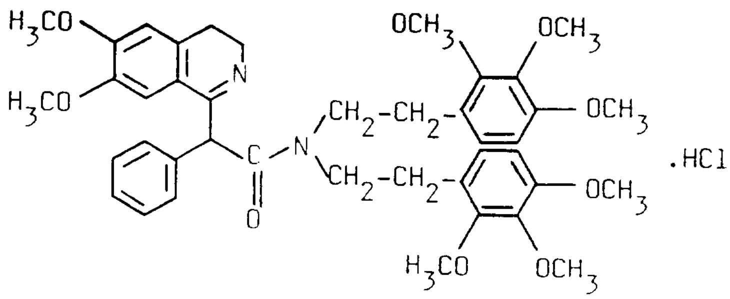

a) 2-(3-4-Dimethoxyphenyl)ethylaminocarbonyl-phenyl-essigsäure-N,N-di-[2-(2,3,4-trimethoxyphenyl)-ethyl]amid

In eine Lösung von 18,0 g (52,4 mmol) Phenylmalonsäuremonoethylester-2-(3,4-dimethoxy-phenyl)ethylamid in 150 ml wasserfreiem Dimethylformamid werden bei Raumtemperatur portionsweise 9,0 g (55,5 mmol) N,N'-Carbonyldiimidazol eingerührt. Nach 30 Min. werden 18,0 g (44,3 mmol) Di-[2-(2,3,4-Trimethoxyphenyl)ethyl)amin zugegeben und 30 Min gerührt. Danach wird das Lösungsmittel im Vakuum abdestilliert, der Rückstand in 1,5 l $CH_2Cl_2$ aufgenommen und nacheinander jeweils 2 mal mit 250 ml Wasser und 200 ml 1N HCl geschüttelt. Die organische Phase wird nach dem Trocknen über $Na_2SO_4$ eingeengt, und der Rückstand nach der Reinigung über eine Kieselgelsäule (Eluens: $CH_2Cl_2$/MeOH 100:2) aus Essigester/Ether kristallisiert.
Ausbeute: 35,5 g

b) 35,0 g (47,5 mmol) Amid (aus Stufe a) und 15 ml (164 mmol) Phosphoroxychlorid werden in 150 ml wasserfreiem Acetonitril 30 Minuten zum Sieden erhitzt. Nach beendeter Umsetzung (Dünnschichtchromatographie-Kontrolle) werden das Lösungsmittel und nicht verbrauchtes Phosphoroxychlorid im Vakuum abdestilliert. Der Rückstand wird mit Eiswasser versetzt, mit Sodalösung alkalisch gestellt und mit ca. 1 l $CH_2Cl_2$ portionsweise extrahiert. Die organische Phase wird mit Wasser gewaschen, über $Na_2SO_4$ getrocknet und eingeengt. Der Rückstand wird zweimal über eine Kieselgelsäule (1. Eluens: $CH_2CL_2$:MeOH 100:2 → 100:4 aufsteigend; 2. Eluens: $CH_2Cl_2$/Essigester 1:1) gereinigt.

Vom gereinigten Produkt (6,5 g) wird durch Lösen in ca. 50 ml Ethanol und Zugabe alkoholischer Salzsäure das Hydrochlorid gebildet. Nach dem Einengen und Trocknen im Hochvakuum bei 50°C verbleiben 11,5 g des gewünschten Produkts. (Fp. 56-64°C, amorph)

Analog können zum Beispiel auch die Verbindungen der Tabelle 12 hergestellt werden.

## Patentansprüche

1. Verwendung von carbocyclisch und heterocyclisch anellierten Dihydropyridinen der Formel

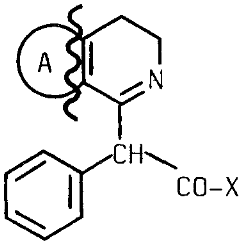

sowie deren tautomere Formen der Formel

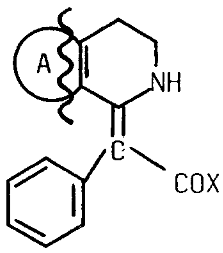

oder

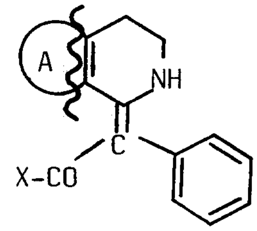

II'

II''

II

in der X für $OR_1$; $NHR_2$; $NR_3R_4$ und

$R_1$ für Wasserstoff, $C_1$-$C_4$-Alkyl; $C_1$-$C_4$-Alkoxyalkyl;

$R_2$ für Wasserstoff, $C_3$-$C_6$-Alkenyl; $C_3$-$C_6$-Alkinyl; $C_3$-$C_6$-Cycloalkyl; $C_3$-$C_6$-Cycloalkenyl; geradkettiges oder verzweigtes $C_1$-$C_6$-Alkyl, welches gewünschtenfalls ein- oder mehrfach mit den nachstehend genannten Substituenten der Gruppen a) bis c) die gleich oder verschieden sein können, substituiert ist:

a) Halogen; Cyano; Hydroxy; Mercapto; $C_1$-$C_4$-Alkoxy; $C_1$-$C_4$-Alkylthio; Amino; Mono-$C_1$-$C_4$-Alkylamino; Di-$C_1$-$C_4$-Alkylamino (wobei die Alkylradikale gleich oder verschieden sein können), Phenoxy (worin die Phenylgruppe wie unter (b) angegeben substituiert sein kann).

b) Phenyl; gewünschtenfalls ein- oder mehrfach gleich oder verschieden substituiert durch die Gruppen Halogen, Trifluormethyl, $C_1$-$C_4$-Alkoxy, Hydroxy, Mercapto, $C_1$-$C_4$-Alkylthio, $C_1$-$C_4$-Alkyl, Amino, Mono-$C_1$-$C_4$-Alkylamino, Di-$C_1$-$C_4$-Alkylamino (wobei die Alkylreste gleich oder verschieden sein können), $C_2$-$C_3$-Acylamino, $C_2$-$C_3$-Acyloxy sowie die vicinal an das Phenylsystem gebundene Gruppe $-O-(CH_2)_n-O-$(mit n = 1 oder 2)

c) ein 5- oder 6-gliedriger gesättigter oder ganz oder teilweise ungesättigter monocyclischer Heterocyclus mit bis zu 3 Heteroatomen aus der Gruppe N, O, S; sowie als bicyclischer Heterocyclus Indol (wobei die zuvor genannen Heterocyclen ein- oder mehrfach durch $C_1$-$C_4$-Alkyl substituiert sein können) $c_3$-$C_6$-Cycloalkyl; $C_5$- oder $C_6$-Cycloalkenyl; $C_2$-$C_3$-Acyl; $C_1$-$C_4$-Alkylsulfonyl; oder Phenyl (welches seinerseits bis zu dreifach wie unter b) beschrieben substituiert sein kann);

oder $R_2$ Phenyl ist, das wie oben unter (b) angegeben substituiert sein kann;

$R_3$ und $R_4$ unabhängig voneinander für $C_1$-$C_4$-Alkyl, welches gewünschtenfalls phenylsubstituiert sein kann, wobei der Phenylsubstituent seinerseits wie vorstehend unter b) substituiert sein kann;

oder

$R_3$ und $R_4$ zusammen mit dem Stickstoffatom, an welches sie gebunden sind, für einen ganz- oder teilweise gesättigten heterocyclischen 5- oder 6-Ring (der außerdem noch bis zu 2 weitere Heteroatome aus der Gruppe N, O, S enthalten kann) bilden, wobei der so erhaltene Heterocyclus durch $C_1$-$C_4$-Alkyl, Hydroxy, oder $(CH_2)_p$-$R_5$ (mit p = 0 oder 1) substituiert sein kann;

und

$R_5$ für ein Phenylradikal, welches gewünschtenfalls wie vorstehend unter b) substituiert ist, steht;

A steht für die anellierten Ringsysteme

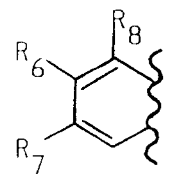

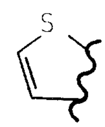

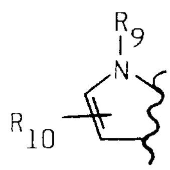

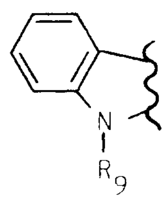

in denen

$R_8$ für Wasserstoff; $C_1$-$C_4$-Alkyl; oder $C_1$-$C_4$-Alkoxy

$R_6$ und $R_7$ die gleich oder verschieden sein können für Wasserstoff; Hydroxy; $C_1$-$C_4$-Alkyl; $C_1$-$C_4$-Alkoxy; Amino; Methansulfonylamino

oder

$R_6$ und $R_7$ gemeinsam für $-O-(CH_2)_n-O-$ (mit n = 1 oder 2)

$R_9$ für Wasserstoff; $C_1$-$C_4$-Alkyl

$R_{10}$ für Wasserstoff; oder 2-Phenyl-2-ethoxycarbonylacetyl;

steht sowie deren Salze mit physiologisch verträglichen Säuren, Basen oder Komplexbildnern für die Herstellung von hirnprotektiven Mitteln.

2. Verwendung von carbocylisch und heterocyclisch anellierten Dihydropyridinen der Formel I bzw. II nach Anspruch 1, in der X für $OR_1$; $NHR_2$; $NR_3R_4$

$R_1$ für Methyl oder Ethyl

$R_2$ für Wasserstoff; geradkettiges oder verzweigtes unsubstituiertes $C_1$-$C_5$-Alkyl; Allyl; Propargyl; $C_3$-$C_6$-Cycloalkyl; 3-Chlorphenyl; 2-Methyl-3-chlorphenyl; oder $C_1$-$C_3$-Alkyl, welches einfach mit einem der in nachstehend genannten Substituenten der Gruppen d) bis f) substituiert ist;

d) Cyano, Hydroxy, Methoxy, Dimethylamino

e) Phenyl, 3,4-Methylendioxyphenyl, durch eine, zwei oder drei Methoxygruppen substituiertes Phenyl, 3-Hydroxy-4-methoxyphenyl,

f) Morpholino, Pyridin-2-yl, Indol-3-yl, Furan-2-yl, Thiophen-2-yl, Pyridin-3-yl, Pyridin-4-yl

$R_3$ und $R_4$ unabhängig voneinander für Methyl; Ethyl; 3-Cyanopropyl; Benzyl; oder 3,4,5-Trimethoxyphenethyl stehen oder

$R_3$ und $R_4$ zusammen mit dem Stickstoffatom an welches sie gebunden sind für Morpholin; Thiomorpholin; Pyrrolidin; Piperazin; 4-Methylpiperazin; 4-Benzylpiperazin; oder 4-(2-Methoxyphenyl)piperazin stehen;

und

A für die anellierten Ringsysteme

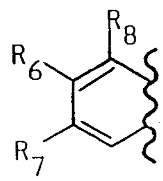

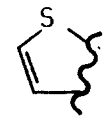

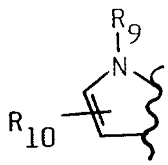

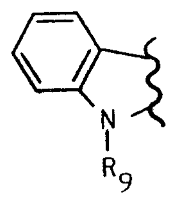

in denen

| | |
|---|---|
| $R_8$ | für Wasserstoff; oder Methoxy |
| $R_6$ | für Methoxy; Hydroxy; Amino; oder Methansulfonylamino |
| $R_7$ | für Wasserstoff; Methoxy; oder Hydroxy |
| $R_9$ | für Methyl |
| $R_{10}$ | für 2-Phenyl-2-ethoxycarbonylacetyl |

steht.

**3.** Verwendung einer Verbindung der Formel I bzw. II nach Anspruch 1 oder 2, worin

A der Rest

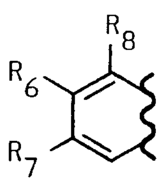

oder

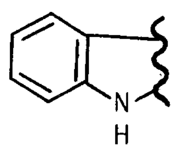

ist.

**4.** Verwendung einer Verbindung der Formel I bzw. II nach einem der Ansprüche 1 bis 3, worin $R_8$ Wasserstoff ist und $R_6$ und $R_7$ unabhängig voneinander Hydroxy oder Methoxy oder gemeinsam $-O-CH_2-O-$ sind, vorzugsweise $R_6$ und $R_7$ Methoxy sind.

**5.** Verwendung einer Verbindung nach einem der Ansprüche 1 bis 4, worin X $-NHR_2$ oder $-NR_3R_4$ ist.

**6.** Verwendung einer Verbindung nach Anspruch 5, worin X $-NHR_2$ ist, worin $R_2$ $C_1$-$C_6$-Alkyl ist.

**7.** Verwendung einer Verbindung nach Anspruch 6, worin $R_2$ $C_1$-$C_4$-Alkyl ist, das unsubstituiert ist oder durch Hydroxy, Phenyl (das durch Hydroxy, Methoxy oder $-O-CH_2-O-$ substituiert sein kann) oder Morpholino substituiert ist.

**8.** Verwendung einer Verbindung nach Anspruch 1, deren Formel

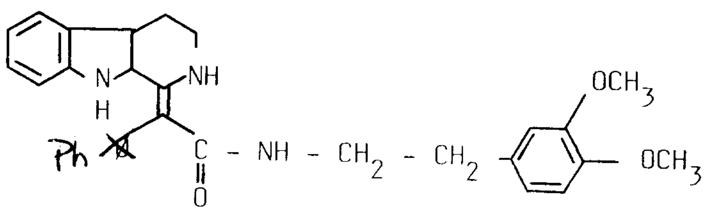

ist, oder deren pharmazeutisch annehmbares Salz.

**9.** Verwendung einer Verbindung nach Anspruch 1, deren Formel

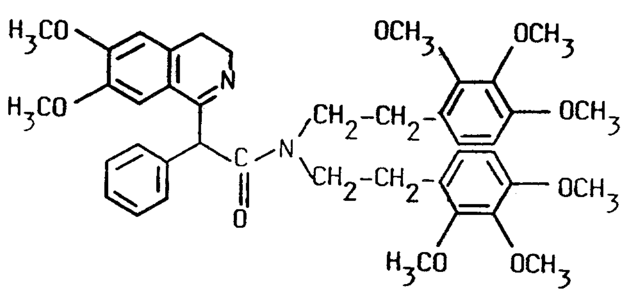

ist, oder deren pharmazeutisch annehmbares Salz.

**10.** 3,4-Dihydroisochinolinderivat der allgemeinen Formel Ie

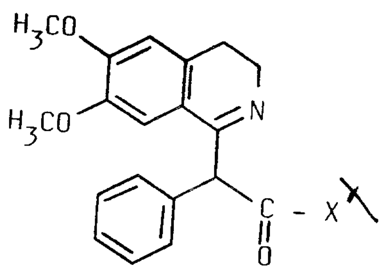

(Ie)

worin X

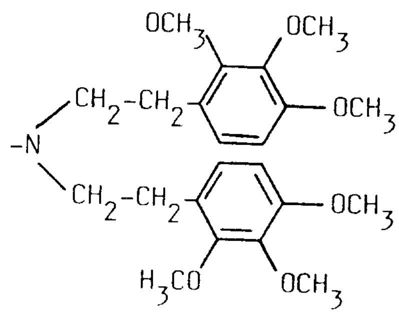

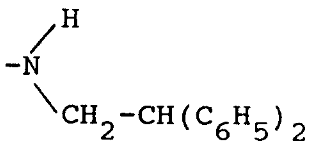

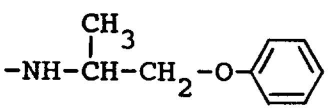

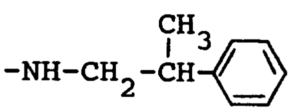

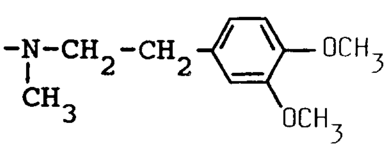

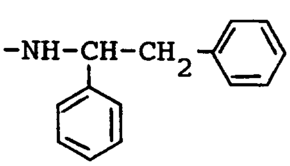

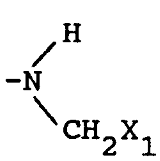

oder

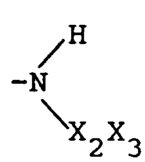

ist,
worin

$X_1$ durch Trifluormethyl oder Ethoxy mono- oder di-substituiertes Phenyl, durch Methoxy und Fluor substituiertes Phenyl oder 2-Methoxy-phenyl ist,

$X_2$ $-CH_2-CH_2-$ oder $-CH_2-CH(CH_3)-$ ist, und

$X_3$ 2,3,4-Trimethoxyphenyl, 2,3-Dimethoxyphenyl, 2,6-Dimethoxyphenyl, 3,6-Dimethoxyphenyl, Thienyl, durch Trifluormethyl oder Ethoxy mono- oder di-substituiertes Phenyl, oder durch Methoxy und Fluor substituiertes Phenyl ist,

oder deren pharmazeutisch annehmbares Salz, vorzugsweise die Verbindung der Formel

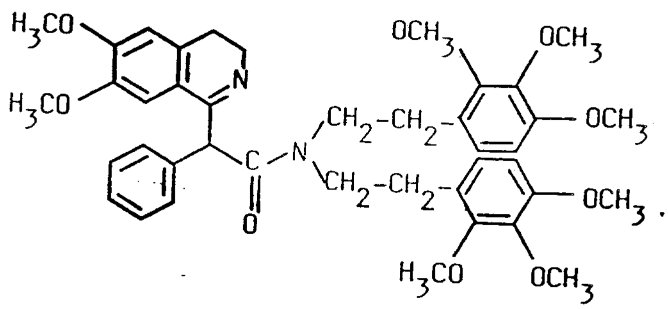

**11.** Verfahren zur Herstellung der Verbindung nach Anspruch 10

a) durch Cyclisierung eines Phenylmalonsäurediamids der Formel III

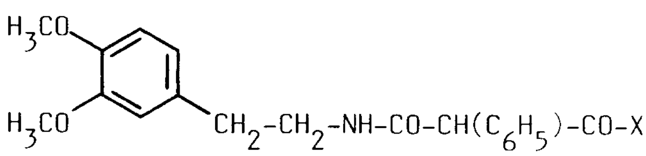

III

in Gegenwart eines Kondensationsmittels oder

b) ausgehend von Carbonsäuren oder Carbonsäurehalogeniden der Formel IV, in der Y für OH oder Halogen steht, durch Umsetzung mit dem Amin der allgemeinen Formel VI

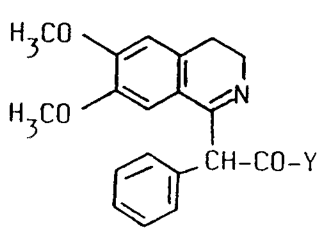

+ XH

IV

VI

in Gegenwart eines geeigneten Kondensationsmittels,
wobei in den obigen Formeln X wie für die Formel Ie definiert ist, und gewünschtenfalls durch Überführen der so erhaltenen Verbindung in ihr pharmazeutisch annehmbares Salz.

**12.** Pharmazeutische Zubereitung enthaltend eine Verbindung gemäß Anspruch 10.

## Claims

**1.** Use of carbocyclically and heterocyclically fused dihydropyridines of formula

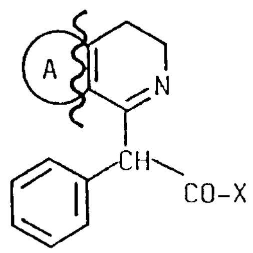

I

and the tautomeric forms thereof of formula

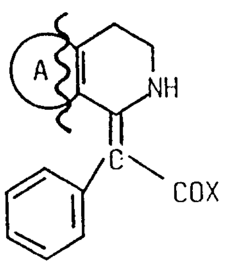

II'

or

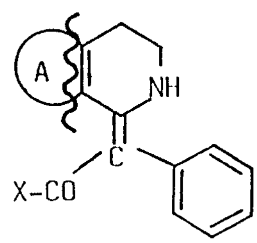

II''

II

wherein X represents $OR_1$; $NHR_2$; $NR_3R_4$
and

$R_1$ represents hydrogen, $C_{1-4}$-alkyl, $C_{1-4}$-alkoxyalkyl

$R_2$ represents hydrogen; $C_{3-6}$-alkenyl, $C_{3-6}$-alkynyl; $C_{3-6}$-cycloalkyl; $C_{3-6}$-cycloalkenyl; straight-chained or branched $C_{1-6}$-alkyl which may if desired be mono- or polysubstituted with the substituents of groups a) to c) listed below, which may be identical or different:

a) halogen; cyano; hydroxy; mercapto; $C_{1-4}$-alkoxy; $C_{1-4}$-alkylthio; amino; mono-$C_{1-4}$-alkylamino; di-$C_{1-4}$-alkylamino (wherein the alkyl radicals may be identical or different), phenoxy (wherein the phenyl ring may be substituted as described in b) below)

b) phenyl; optionally mono- or polysubstituted, by identical or different substituents, by the groups halogen, trifluoromethyl, $C_{1-4}$-alkoxy, hydroxy, mercapto, $C_{1-4}$-alkylthio, $C_{1-4}$-alkyl, amino, mono-$C_{1-4}$- alkylamino, di-$C_{1-4}$-alkylamino (wherein the alkyl groups may be identical or different), $C_{2-3}$- acylamino, $C_{2-3}$-acyloxy and the group $-O-(CH_2)_n-O$ vicinally bound to the phenyl system (wherein n = 1 or 2)

c) a 5- or 6-membered saturated or wholly or partially unsaturated monocyclic heterocyclic group having up to 3 heteroatoms selected from the group N, O, S; and as a bicyclic heterocycle indole

(whilst the above-mentioned heterocycles may be mono- or polysubstituted by $C_{1-4}$-alkyl), $C_{3-6}$-cycloalkyl; $C_5$- or $C_6$-cycloalkenyl; $C_{2-3}$-acyl; $C_{1-4}$-alkylsulphonyl; or phenyl (which may in turn be substituted up to three times as described in b));

or $R_2$ represents phenyl, which may be substituted as described in b) above;

$R_3$ and $R_4$ independently of each other represent $C_{1-4}$-alkyl, which may if desired be phenyl-substituted, whilst the phenyl substituent may in turn be substituted as under b) hereinbefore;

or

$R_3$ and $R_4$ together with the nitrogen atom to which they are bound represent a wholly or partially saturated heterocyclic 5- or 6-membered ring (which may also contain up to 2 further heteroatoms from the group N, O, S), whilst the heterocyclic group thus obtained may be substituted by $C_{1-4}$-alkyl, hydroxy or $(CH_2)_p$-$R_5$ (where p = 0 or 1)

and

$R_5$ represents a phenyl radical which is optionally substituted as under b) hereinbefore;

A represents the fused ring systems

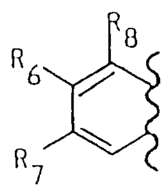

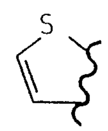

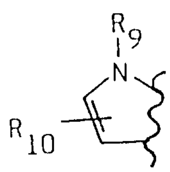

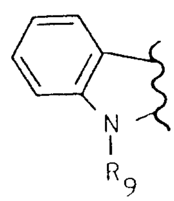

wherein

$R_8$ represents hydrogen; $C_{1-4}$-alkyl or $C_{1-4}$-alkoxy

$R_6$ and $R_7$ which may be identical or different represent hydrogen; hydroxy; $C_{1-4}$-alkyl; $C_{1-4}$-alkoxy; amino; methanesulphonylamino

or

$R_6$ and $R_7$ together represent -O-$(CH_2)_n$-O- (where n = 1 or 2)

$R_9$ represents hydrogen; $C_{1-4}$-alkyl

$R_{10}$ represents hydrogen or 2-phenyl-2-ethoxycarbonyl-acetyl;

and the salts thereof with physiologically acceptable acids, bases or complex-forming agents for the preparation of cerebroprotective agents.

**2.** Use of carbocyclically and heterocyclically fused dihydropyridines of formula I or II according to claim 1, wherein X represents $OR_1$; $NHR_2$; $NR_3R_4$

$R_1$ represents methyl or ethyl

$R_2$ represents hydrogen; straight-chained or branched unsubstituted $C_{1-5}$-alkyl; allyl; propargyl; $C_{3-6}$-cycloalkyl; 3-chlorophenyl; 2-methyl-3-chlorophenyl; or $C_{1-3}$-alkyl, which is mono-substituted with

one of the substituents of groups d) to f) listed hereinafter;

d) cyano, hydroxy, methoxy, dimethylamino

e) phenyl, 3,4-methylenedioxyphenyl, mono-, di- or tri-methoxy substituted phenyl, 3-hydroxy-4-methoxyphenyl,

f) morpholino, pyridin-2-yl, indol-3-yl, furan-2-yl, thiophen-2-yl, pyridin-3-yl, pyridin-4-yl

$R_3$ and $R_4$ independently of each other represent methyl; ethyl; 3-cyanopropyl; benzyl; or 3,4,5-trimethoxyphenethyl or

$R_3$ and $R_4$ together with the nitrogen atom to which they are bound represent morpholine; thiomorpholine; pyrrolidine; piperazine; 4-methylpiperazine; 4-benzylpiperazine; or 4-(2-methoxyphenyl)piperazine;

and

A represents the fused ring systems

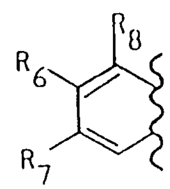

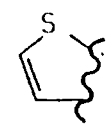

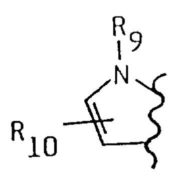

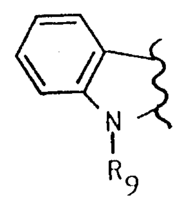

wherein

$R_8$ represents hydrogen or methoxy

$R_6$ represents methoxy; hydroxy; amino; or methanesulphonylamino

$R_7$ represents hydrogen; methoxy; or hydroxy

$R_9$ represents methyl

$R_{10}$ represents 2-phenyl-2-ethoxycarbonylacetyl.

**3.** Use of a compound of formula I or II according to claim 1 or 2, wherein

A is the group

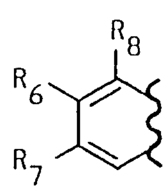

or

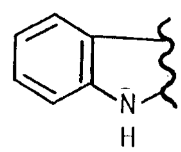

**4.** Use of a compound of formula I or II according to one of claims 1 to 3, wherein $R_8$ is hydrogen and $R_6$ and $R_7$ inde-

pendently of one another represent hydroxy or methoxy or together represent -O-$CH_2$-O-, $R_6$ and $R_7$ preferably representing methoxy.

5. Use of a compound according to one of claims 1 to 4, wherein X is -$NHR_2$ or -$NR_3R_4$.

6. Use of a compound according to claim 5, wherein X is $NHR_2$, wherein $R_2$ represents $C_{1-6}$-alkyl.

7. Use of a compound according to claim 6, wherein $R_2$ is $C_{1-4}$-alkyl which is unsubstituted or substituted by hydroxy, phenyl, (which may be substituted by hydroxy, methoxy or -O-$CH_2$-O-) or morpholino.

8. Use of a compound according to claim 1, of the formula

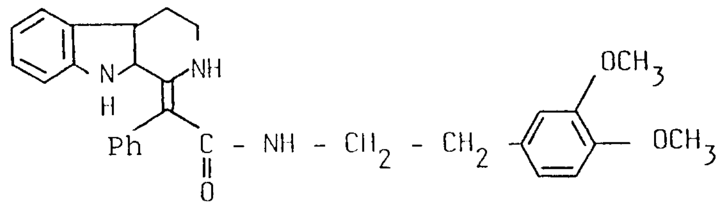

or a pharmaceutically acceptable salt thereof.

9. Use of a compound according to claim 1, of the formula

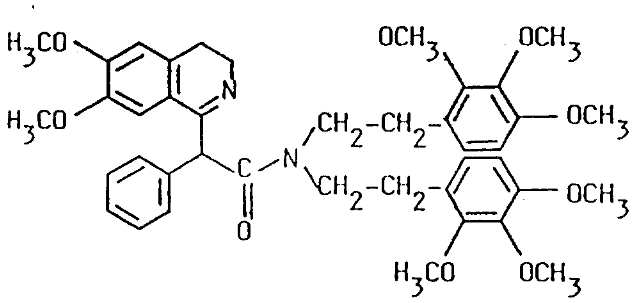

or a pharmaceutically acceptable salt thereof.

10. 3,4-dihydroisoquinoline derivatives of general formula Ie

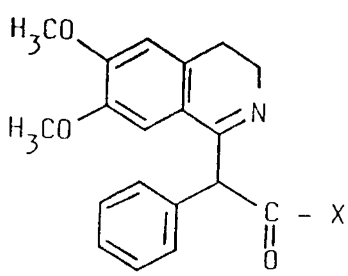

Ie

wherein X represents a group

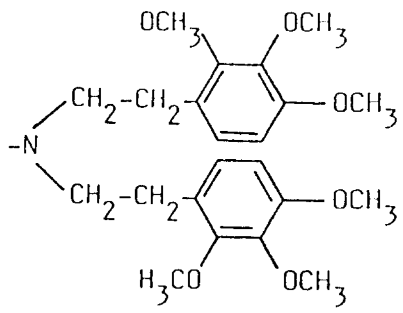

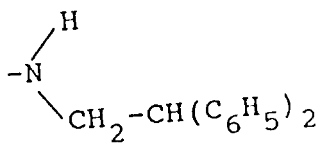

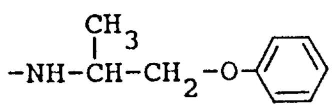

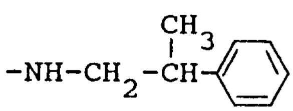

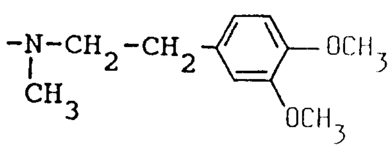

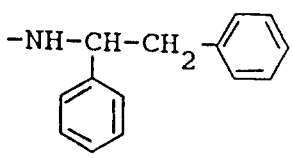

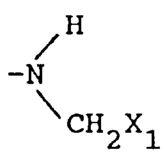

or

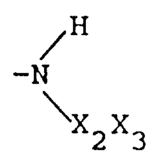

in which

$X_1$ represents a phenyl group, mono- or di-substituted by a trifluoromethyl or ethoxy group, a phenyl group or a 2-methoxyphenyl group substituted by a fluorine atom and a methoxy group;

$X_2$ represents a group $-CH_2-CH_2-$ or $-CH_2-CH(CH_3)-$; and

$X_3$ represents a 2,3,4-trimethoxyphenyl, 2,3-dimethoxyphenyl, 2,6-dimethoxyphenyl, 3,6-dimethoxyphenyl or thienyl group, a phenyl group mono- or di-substituted by a trifluoromethyl or ethoxy group or a phenyl group substituted by a methoxy group and a fluorine atom

or their pharmaceutically acceptable salts, preferably a compound of formula

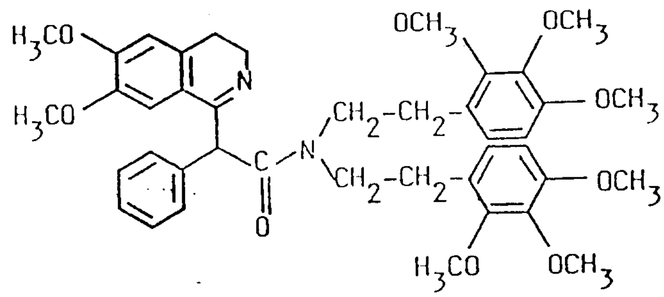

**11.** Process for preparing the compound according to claim 10

a) by cyclising a phenylmalonic acid diamide of formula III

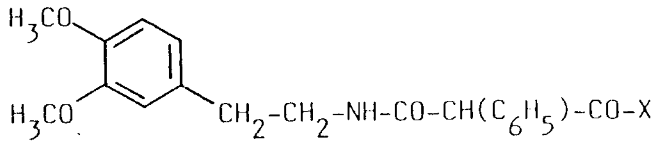

III

in the presence of a condensing agent or

b) starting from carboxylic acids or carboxylic acid halides of formula IV, wherein Y represents OH or halogen, by reacting with the amine of general formula VI

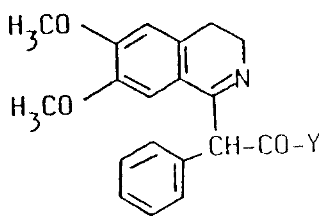

IV

+ XH

VI

in the presence of a suitable condensing agent,
whilst in the above formulae X is as defined for formula Ie,
and, if desired, converting the compound thus obtained into a pharmaceutically acceptable salt thereof.

**12.** Pharmaceutical preparation containing a compound according to claim 10.

## Revendications

**1.** Utilisation de dihydropyridines carbocycliquement et hétérocycliquement condensées de formule

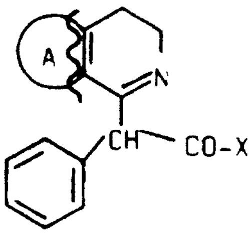

I

et de leurs formes tautomères de formule

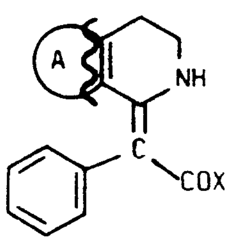

ou

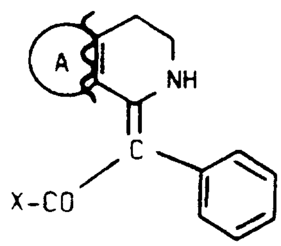

II' II''

II

où X représente $OR_1$, $NHR_2$, $NR_3R_4$
et

$R_1$ représente hydrogène, alkyle en $C_1$-$C_4$, alcoxyalkyle en $C_1$-$C_4$,

$R_2$ représente hydrogène, alcényle en $C_3$-$C_6$, alcynyle en $C_3$-$C_6$, cycloalkyle en $C_3$-$C_6$, cycloalcényle en $C_3$-$C_6$, alkyle en $C_1$-$C_6$ linéaire ou ramifié qui est substitué si on le souhaite une ou plusieurs fois par les substituants des groupes a) à c) cités dans la suite qui peuvent être identiques ou différents:

a) halogène, cyano, hydroxyle, mercapto, alcoxy en $C_1$-$C_4$, alkylthio en $C_1$-$C_4$, amino, monoalkylamino en $C_1$-$C_4$, dialkylamino en $C_1$-$C_4$ (ou les radicaux alkyle peuvent être identiques ou différents), phénoxy (où le groupe phényle peut être substitué comme indiqué en (b);
b) phényle, si on le souhaite substitué une ou plusieurs fois de manière identique ou différente par les groupes halogène, trifluorométhyle, alcoxy en $C_1$-$C_4$, hydroxyle, mercapto, alkylthio en $C_1$-$C_4$, alkyle en $C_1$-$C_4$, amino, monoalkylamino en $C_1$-$C_4$, dialkylamino en $C_1$-$C_4$ (où les restes alkyle peuvent être identiques ou différents), acylamino en $C_2$-$C_3$, acyloxy en $C_2$-$C_3$ et le groupe $-O-(CH_2)_n-O-$ (avec n = 1 ou 2) lié de manière vicinale au système phényle;
c) un hétérocycle monocyclique saturé ou partiellement ou totalement insaturé à 5 ou 6 chaînons ayant jusqu'à 3 hétéroatomes du groupe de N, O, S; ainsi que, comme hétérocycle bicyclique, l'indole (les hétérocycles cités précédemment pouvant être substitués une ou plusieurs fois par alkyle en $C_1$-$C_4$), cycloalkyle en $C_3$-$C_6$, cycloalcényle en $C_5$ ou $C_6$, acyle en $C_2$-$C_3$, alkylsulfonyle en $C_1$-$C_4$ ou phényle (qui, pour sa part, peut être substitué jusqu'à trois fois comme décrit en b));

ou $R_2$ est phényle qui peut être substitué comme indiqué plus haut en (b);

$R_3$ et $R_4$ représentent indépendamment l'un de l'autre alkyle en $C_1$-$C_4$, qui peut, si on le souhaite, être substitué par phényle, le substituant phényle pouvant pour sa part être substitué comme précédemment en b);

ou

$R_3$ et $R_4$ forment avec l'atome d'azote auquel ils sont liés un cycle hétérocyclique à 5 ou 6 chaînons totalement ou partiellement saturé (qui peut contenir en outre encore jusqu'à 2 autres hétéroatomes du groupe de N, O, S), l'hétérocycle ainsi obtenu pouvant être substitué par alkyle en $C_1$-$C_4$, hydroxyle ou $(CH_2)_p$-$R_5$ (avec p = 0 ou 1);

et

$R_5$ représente un radical phényle qui, si on le souhaite, est substitué comme précédemment en b);
A représente les systèmes cycliques condensés

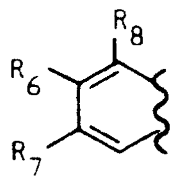

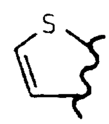

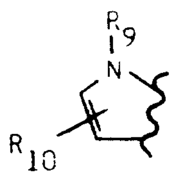

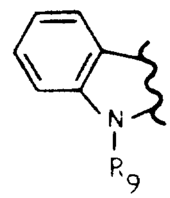

dans lesquels

$R_8$ représente l'hydrogène, alkyle en $C_1$-$C_4$ ou alcoxy en $C_1$-$C_4$,
$R_6$ et $R_7$ qui peuvent être identiques ou différents représentent l'hydrogène, hydroxyle, alkyle en $C_1$-$C_4$, alcoxy en $C_1$-$C_4$, amino, méthanesulfonylamino

ou

$R_6$ et $R_7$ représentent ensemble $-O-(CH_2)_n-O-$ (avec n = 1 ou 2)
$R_9$ représente l'hydrogène, alkyle en $C_1$-$C_4$,
$R_{10}$ représente l'hydrogène, ou 2-phényl-2-éthoxycarbonylacétyle,

ainsi que de leurs sels avec des acides, bases ou complexants physiologiquement acceptables pour la préparation d'agents cérébroprotecteurs.

**2.** Utilisation de dihydropyridines carbocycliquement et hétérocycliquement condensées de formule I ou II selon la revendication 1
où X représente $OR_1$, $NHR_2$, $NR_3R_4$,

$R_1$ représente méthyle ou éthyle,
$R_2$ représente hydrogène, alkyle en $C_1$-$C_5$ linéaire ou ramifié non substitué, allyle, propargyle, cycloalkyle en $C_3$-$C_6$, 3-chlorophényle, 2-méthyl-3-chlorophényle ou alkyle en $C_1$-$C_3$ qui est substitué une fois par l'un des substituants des groupes d) à f) cités dans la suite:

d) cyano, hydroxyle, méthoxy, diméthylamino,
e) phényle, 3,4-méthylènedioxyphényle, phényle substitué par un, deux ou trois groupes méthoxy, 3-hydroxy-4-méthoxyphényle,
f) morpholino, pyridin-2-yle, indol-3-yle, furan-2-yle, thiophén-2-yle, pyridin-3-yle, pyridin-4-yle,

$R_3$ et $R_4$ représentent indépendamment l'un de l'autre méthyle, éthyle, 3-cyanopropyle, benzyle ou 3,4,5-trimé-

thoxy-phénéthyle ou

ou

$R_3$ et $R_4$ représentent avec l'atome d'azote auquel ils sont liés morpholine, thiomorpholine, pyrrolidine, pipérazine, 4-méthylpipérazine, 4-benzylpipérazine ou 4-(2-méthoxyphényl)pipérazine;

et

A représente les systèmes cycliques condensés

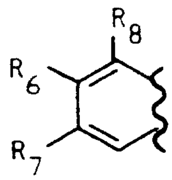
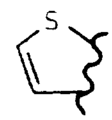
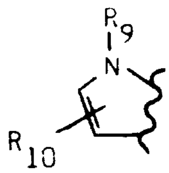
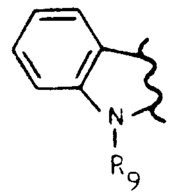

dans lesquels

$R_8$ représente l'hydrogène ou méthoxy,
$R_6$ représente méthoxy, hydroxyle, amino ou méthanesulfonylamino,
$R_7$ représente hydrogène, méthoxy ou hydroxyle,
$R_9$ représente méthyle,
$R_{10}$ représente 2-phényl-2-éthoxycarbonylacétyle.

**3.** Utilisation d'un composé de formule I ou II selon la revendication 1 ou 2, où

A est le reste

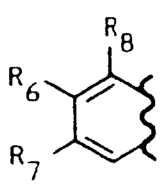

ou

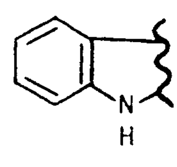

**4.** Utilisation d'un composé de formule I ou II selon l'une des revendications 1 à 3, où $R_8$ est l'hydrogène et $R_6$ et $R_7$ sont indépendamment l'un de l'autre hydroxyle ou méthoxy ou sont ensemble $-O-CH_2-O-$, de préférence $R_6$ et $R_7$ sont méthoxy.

**5.** Utilisation d'un composé selon l'une des revendications 1 à 4 où X est $-NHR_2$ ou $-NR_3R_4$.

**6.** Utilisation d'un composé selon la revendication 5 où X est $-NHR_2$ où $R_2$ est alkyle en $C_1$-$C_6$.

7. Utilisation d'un composé selon la revendication 6 où $R_2$ est alkyle en $C_1$-$C_4$ qui est non substitué ou qui est substitué par hydroxyle, phényle (qui peut être substitué par hydroxyle, méthoxy ou -O-$CH_2$-O-) ou morpholino.

8. Utilisation d'un composé selon la revendication 1 dont la formule est

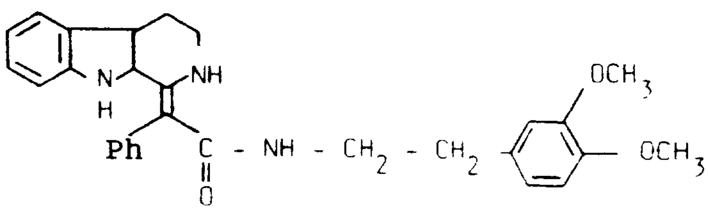

ou de son sel pharmaceutiquement acceptable.

9. Utilisation d'un composé selon la revendication 1 dont la formule est

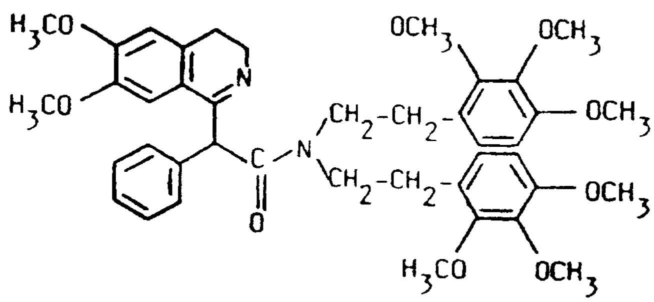

ou de son sel pharmaceutiquement acceptable.

10. Dérivé de 3,4-dihydroisoquinoléine de formule générale Ie

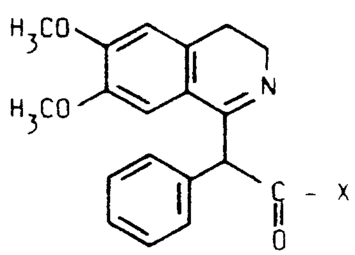

(Ie)

où X est

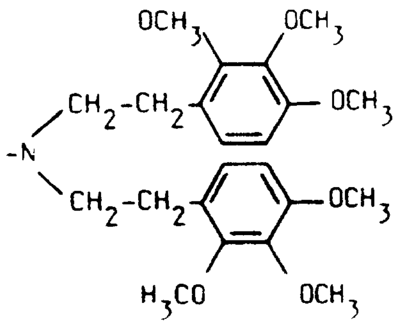

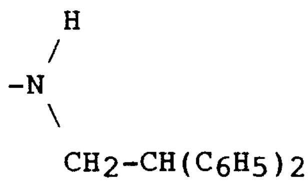

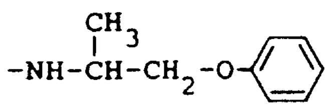

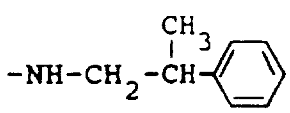

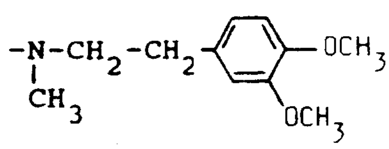

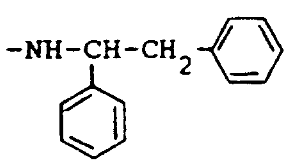

```
      H                       H
     /                       /
   -N            ou        -N
     \                       \
      CH2-X1                  X2X3
```

où

$X_1$ est phényle mono- ou disubstitué par trifluorométhyle ou éthoxy, phényle substitué par méthoxy et fluor ou 2-méthoxyphényle,

$X_2$ est $-CH_2-CH_2-$ ou $-CH_2-CH(CH_3)-$

et

$X_3$ est 2,3,4-triméthoxyphényle, 2,3-diméthoxyphényle, 2,6-diméthoxyphényle, 3,6-diméthoxyphényle, thiényle, phényle mono- ou disubstitué par trifluorométhyle ou éthoxy ou phényle substitué par méthoxy et fluor,

ou son sel pharmaceutiquement acceptable,
de préférence le composé de formule

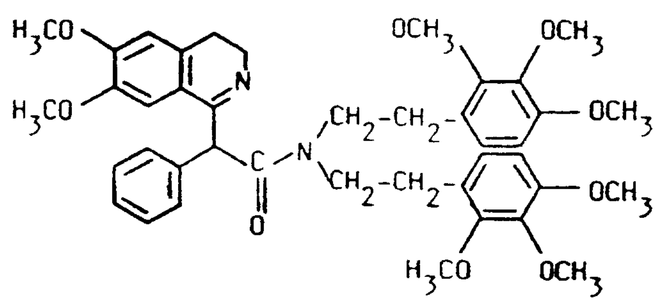

**11.** Procédé de préparation du composé selon la revendication 10

a) par cyclisation d'un diamide de l'acide phénylmalonique de formule III

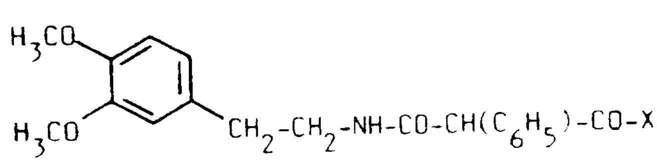

III

en présence d'un agent de condensation
ou
b) à partir d'acides carboxyliques ou d'halogénures d'acides carboxyliques de formule IV où Y représente OH ou halogène, par réaction avec l'amine de formule générale VI

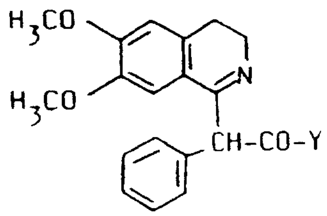

+ XH

IV VI

en présence d'un agent de condensation approprié où dans les formules ci-dessus X est défini comme pour la formule Ie, et, si on le souhaite, par conversion du composé ainsi obtenu en son sel pharmaceutiquement acceptable.

**12.** Préparation pharmaceutique contenant un composé selon la revendication 10.